# EUROPEAN PATENT APPLICATION

(11) **EP 4 458 806 A1**
(43) Date of publication of application: **06.11.2024**
(21) Application number: 22914988.5
(22) Date of filing: 29.12.2022
(51) Int. Cl.: C07C 229/12, C07C 229/16, C07C 217/08, A61K 31/7088, A61K 47/18, A61K 9/127

(54) **LIPID AND COMPOSITION USED FOR DELIVERY**

(30) Priority: 29.12.2021 CN 202111638123; 09.06.2022 CN 202210651178
(71) Applicant: Jiangsu Hengrui Pharmaceuticals Co., Ltd., Lianyungang, Jiangsu 222047 (CN); Shanghai Senhui Medicine Co., Ltd., Shanghai 201203 (CN); Shanghai Shengdi Pharmaceutical Co., Ltd, Shanghai 201210 (CN)
(72) Inventor: ZHU, Lingjian, Shanghai 201203 (CN); SHI, Jianyu, Shanghai 201203 (CN); LIU, Chongyi, Shanghai 201210 (CN); JIANG, Jun, Shanghai 201210 (CN); HUANG, Jian, Shanghai 201203 (CN)
(74) Representative: Potter Clarkson
(86) International application number: PCT/CN2022/143074
(87) International publication number: WO 2023/125738

(57) **Abstract**

Provided are a lipid and a composition used for delivery. Specifically provided is a compound represented by formula I or a salt thereof, wherein R¹ to R³, H¹ to H³, L¹, and L² are as defined in the description.

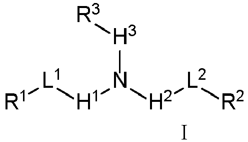

## Description

### TECHNICAL FIELD

The disclosure pertains to the field of pharmaceutics and relates to a lipid and a composition for delivery.

### BACKGROUND

Nucleic acid-based drugs, such as messenger RNA (mRNA), antisense oligonucleotides, small interfering RNA (siRNA), and plasmids, have vast potential for application. A problem that hinders the advancement of this technology is how to safely and effectively deliver these drugs to target organs and cells within the body.

Currently, nucleic acid drug delivery systems are categorized into 2 main types: viral vector systems and non-viral systems. Lipid nanoparticle-mediated nucleic acid drug delivery is a primary method for non-viral systems.

In gene therapy and vaccine applications, lipid nanoparticles have been shown to be excellent vectors for nucleic acids for treating various diseases. Lipid nanoparticles formed of cationic lipids and other auxiliary lipids such as cholesterol, phospholipids, and PEGylated lipids encapsulate nucleic acids, protecting them from degradation and promoting cellular uptake, reducing immune responses. In addition, lipid nanoparticles offer other advantages for the delivery of bioactive ingredients into cells, including good targeting, minimal side effects, good stability, efficient transfection, etc.

The rapid development in the field of nucleic acid molecule-based therapy has generated an increased demand for the delivery of nucleic acid drugs. Therefore, there is a need to develop efficient and safe nucleic acid delivery vectors.

### SUMMARY

The disclosure provides a compound represented by formula I or a salt thereof, 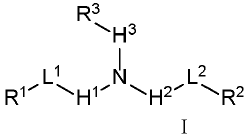
wherein L¹ and L² are each independently selected from the group consisting of -C(O)O-, -OC(O)-, -C(O)-, -OC(O)O-, -O-, -S(O)ₓ-, -S-S-, -C(O)S-, -SC(O)-, -NR^{a}C(O)-, -C(O)NR^{a}-, -NR^{a}C(O)NR^{a}-, -NR^{a}C(O)O-, -OC(O)NR^{a}-, or a bond, and R^{a} is selected from the group consisting of hydrogen or C₁₋₆ alkyl or C₂₋₆ alkenyl;
H¹ and H² are each independently selected from the group consisting of C₁₋₁₂ heteroalkylene, C₁₋₁₂ alkylene, and C₂₋₁₂ alkenylene, and at least one of H¹ and H² is C₁₋₁₂ heteroalkylene;
H³ is selected from the group consisting of C₁₋₂₄ alkylene, C₂₋₂₄ alkenylene, C₃₋₈ cycloalkylene, or C₃₋₈ cycloalkenylene;
R¹ and R² are each independently selected from the group consisting of C₁₋₂₄ alkyl or C₂₋₂₄ alkenyl;
R³ is selected from the group consisting of hydrogen, -CN, -C(O)OR⁴, -OC(O)R⁴, -OR⁵, or -NR⁵C(O)R⁴; R⁴ is selected from the group consisting of C₁₋₆ alkyl or C₂₋₆ alkenyl; R⁵ is selected from the group consisting of hydrogen, C₁₋₆ alkyl, or C₂₋₆ alkenyl;
x is selected from the group consisting of 0, 1, or 2.

In some embodiments, at least one of H¹ and H² in the compound represented by formula I or the salt thereof is heteroalkylene comprising at least one heteroatom selected from the group consisting of O, N, and S.

In some embodiments, H¹ in the compound represented by formula I or the salt thereof is selected from the group consisting of C₁₋₁₂ heteroalkylene, preferably C₂₋₉ heteroalkylene. In some embodiments, the heteroalkylene is heteroalkylene comprising at least one oxygen atom. In some embodiments, the heteroalkylene is heteroalkylene comprising two oxygen atoms. In some embodiments, the heteroalkylene is heteroalkylene comprising at least one nitrogen atom. In some embodiments, the heteroalkylene is heteroalkylene comprising at least one oxygen atom.

In some embodiments, L¹ and L² in the compound represented by formula I or the salt thereof are each independently selected from the group consisting of -C(O)O-, -OC(O)-, or a bond.

In some embodiments, L¹ and L² in the compound represented by formula I or the salt thereof are each independently selected from the group consisting of -C(O)-, -OC(O)O-, -O-, or a bond.

In some embodiments, L¹ and L² in the compound represented by formula I or the salt thereof are each independently selected from the group consisting of -S(O)ₓ-, -S-S-, -C(O)S-, -SC(O)-, or a bond.

In some embodiments, L¹ and L² in the compound represented by formula I or the salt thereof are each independently selected from the group consisting of -NR^{a}C(O)-, -C(O)NR^{a}-, -NR^{a}C(O)NR^{a}-, -NR^{a}C(O)O-, or a bond.

In some embodiments, L¹ and L² in the compound represented by formula I or the salt thereof are each independently selected from the group consisting of -NR^{a}C(O)NR^{a}-, -NR^{a}C(O)O-, -OC(O)NR^{a}-, or a bond.

In another aspect, in some embodiments, R¹ and R² in the compound represented by formula I or the salt thereof are each independently selected from the group consisting of C₂₋₂₄ alkyl (including, but not limited to, C₂ alkyl, C₃ alkyl, C₄ alkyl, C₅ alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, and C₂₁ alkyl). In some other embodiments, R¹ and R² in the compound represented by formula I or the salt thereof are each independently selected from the group consisting of C₄-₁₈ alkyl.

In some embodiments, R¹ and R² in the compound represented by formula I or the salt thereof are each independently selected from the group consisting of branched-chain C₄-₁₈ alkyl.

In some embodiments, R¹ and R² in the compound represented by formula I or the salt thereof are each independently selected from the group consisting of straight-chain C₄-₁₈ alkyl.

In some embodiments, R¹ in the compound represented by formula I or the salt thereof is selected from the group consisting of straight-chain C₄-₁₈ alkyl, and R² is selected from the group consisting of branched-chain C₄-₁₈ alkyl.

In some embodiments, R¹ in the compound represented by formula I or the salt thereof is selected from the group consisting of branched-chain C₄-₁₈ alkyl, and R² is selected from the group consisting of branched-chain C₄-₁₈ alkyl.

R¹ and R² in the compound represented by formula I or the salt thereof provided in some embodiments are each independently selected from the group consisting of C₂₋₂₄ alkenyl (including, but not limited to, C₂ alkenyl, C₃ alkenyl, C₄ alkenyl, C₅ alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, C₂₀ alkenyl, and C₂₁ alkenyl). R¹ and R² in the compound represented by formula I or the salt thereof provided in some embodiments are each independently selected from the group consisting of C₄-₁₈ alkenyl.

In some embodiments, R¹ and R² in the compound represented by formula I or the salt thereof are each independently selected from the group consisting of branched-chain C₄-₁₈ alkenyl.

In some embodiments, R¹ and R² in the compound represented by formula I or the salt thereof are each independently selected from the group consisting of straight-chain C₄-₁₈ alkenyl.

In some embodiments, R¹ in the compound represented by formula I or the salt thereof is selected from the group consisting of straight-chain C₄-₁₈ alkenyl, and R² is selected from the group consisting of branched-chain C₄-₁₈ alkenyl.

In some embodiments, R¹ in the compound represented by formula I or the salt thereof is selected from the group consisting of branched-chain C₄-₁₈ alkenyl, and R² is selected from the group consisting of branched-chain C₄-₁₈ alkenyl.

Further, the compound represented by formula I provided in some embodiments is 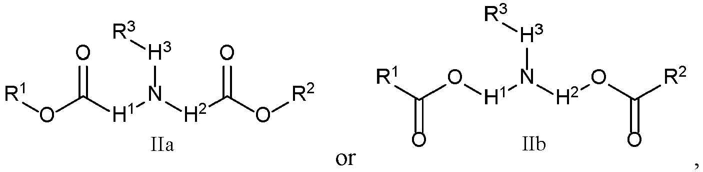 wherein H¹, H², H³, R¹, R², and R³ are as previously defined.

In some embodiments, H³ in the compound represented by formula I or IIa or IIb or the salt thereof is selected from the group consisting of C₂₋₂₄ alkenylene, C₃₋₈ cycloalkylene, or C₃₋₈ cycloalkenylene; or H³ is selected from the group consisting of C₁₋₂₄ alkylene. In some other embodiments, H³ in the compound represented by formula I or IIa or IIb or the salt thereof is selected from the group consisting of C₃₋₈ cycloalkylene.

In some embodiments, C₂₋₂₄ alkenylene includes, but is not limited to, C₂ alkenylene, C₃ alkenylene, C₄ alkenylene, C₅ alkenylene, C₆ alkenylene, C₇ alkenylene, C₈ alkenylene, C₉ alkenylene, C₁₀ alkenylene, C₁₁ alkenylene, C₁₂ alkenylene, C₁₃ alkenylene, C₁₄ alkenylene, C₁₅ alkenylene, C₁₆ alkenylene, C₁₇ alkenylene, C₁₈ alkenylene, C₁₉ alkenylene, C₂₀ alkenylene, C₂₁ alkenylene, C₂₂ alkenylene, or C₂₃ alkenylene.

In some embodiments, C₂₋₂₄ alkylene includes, but is not limited to, C₄ alkylene, C₅ alkylene, C₆ alkylene, C₇ alkylene, C₈ alkylene, C₉ alkylene, C₁₀ alkylene, C₁₁ alkylene, C₁₂ alkylene, C₁₃ alkylene, C₁₄ alkylene, C₁₅ alkylene, C₁₆ alkylene, C₁₇ alkylene, C₁₈ alkylene, C₁₉ alkylene, C₂₀ alkylene, C₂₁ alkylene, C₂₂ alkylene, or C₂₃ alkylene.

The compound represented by formula I or IIa or IIb provided in some other embodiments is 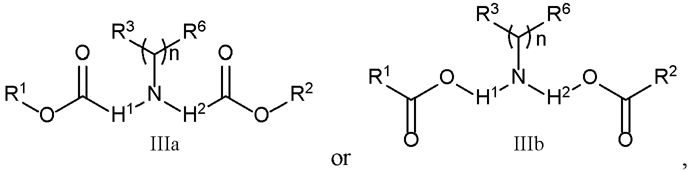 wherein each R⁶ is independently selected from the group consisting of hydrogen, hydroxy, C₁₋₆ alkyl, or C₂₋₆ alkenyl; n is selected from the group consisting of integers between 1 and 12 (including, but not limited to, 2, 3, 4, 5, 6, 7, 8, 9, 10, 11, and 12); H¹, H², R¹, R², and R³ are as defined in the compound represented by formula I.

In some embodiments, n in the compound represented by formula IIIa or IIIb or the salt thereof is 2, 3, 4, 5, or 6.

In some embodiments, R³ in the compound represented by formula I or formula IIIa or IIIb or the salt thereof is selected from the group consisting of -CN or hydroxy. In some embodiments, R³ in the compound represented by formula I or formula IIIa or IIIb or the salt thereof is selected from the group consisting of -C(O)OR⁴, -OC(O)R⁴, or -NHC(O)R⁴, and R⁴ is as previously defined.

In some embodiments, R⁴ in the compound represented by formula I or the salt thereof is selected from the group consisting of C₁₋₆ alkyl, including, but not limited to, methyl, ethyl, or propyl.

In some embodiments, the compound represented by formula I is 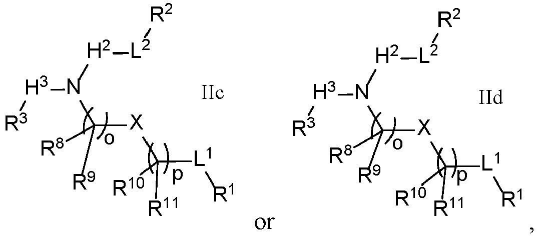 wherein X is -N(R⁷)- or -O-; o is selected from the group consisting of integers between 1 and 11, p is selected from the group consisting of integers between 1 and 5, and o + p is not greater than 12; R⁷ is selected from the group consisting of hydrogen, C₁₋₆ alkyl, or C₂₋₆ alkenyl; R⁸, R⁹, R¹⁰, and R¹¹ are each independently selected from the group consisting of hydrogen, hydroxy, C₁₋₆ alkyl, or C₂₋₆ alkenyl; L¹, L², H², H³, R¹, R², and R³ are as defined in the compound represented by formula I.

In some embodiments, p in the compound represented by formula IIc or IId or the salt thereof is selected from the group consisting of 1 or 2.

In some embodiments, o in the compound represented by formula IIc or IId or the salt thereof is selected from the group consisting of 4, 5, 6, 7, or 8.

In some embodiments, X in the compound represented by formula IIc or IId or the salt thereof is -O-.

In some embodiments, R⁸, R⁹, R¹⁰, and R¹¹ in the compound represented by formula IIc or IId or the salt thereof are each independently selected from the group consisting of hydrogen.

In some embodiments, R¹⁰ and R¹¹ in the compound represented by formula IIc or IId or the salt thereof are each independently selected from the group consisting of C₁₋₆ alkyl. In some embodiments, R³ in the compound represented by formula IIc or IId or the salt thereof is selected from the group consisting of hydroxy.

In some other embodiments, the compound represented by formula I is 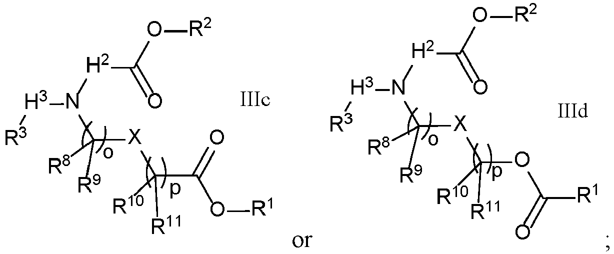 H², R¹, R², and R³ are as defined in the compound represented by formula I; R⁸, R⁹, R¹⁰, R¹¹, X, o, and p are as defined in the compound represented by formula IIc.

In some embodiments, H² in the compound represented by formula I or IIIc or IIId or the salt thereof is selected from the group consisting of C₃₋₇ alkylene or C₃₋₇ alkenylene. The compound represented by formula I provided in some embodiments is 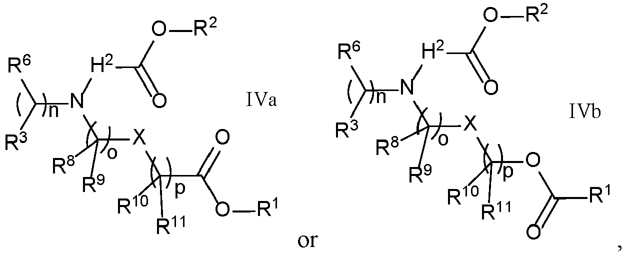 wherein H², R¹, R², and R³ are as defined in the compound represented by formula I; R⁸, R⁹, R¹⁰, R¹¹, X, o, and p are as defined in the compound represented by formula IIc; R⁶ and n are as previously defined.

In another aspect, in some embodiments, R¹ and R² in the compound represented by formula I or the salt thereof each have the following structure: 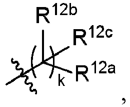 wherein R^{12a}, R^{12b}, and R^{12c} are each independently selected from the group consisting of hydrogen, C₁₋₁₂ alkyl, or C₂₋₁₂ alkenyl, and k is an integer between 2 and 12.

In some embodiments, R^{12c} in the compound represented by formula I or the salt thereof is selected from the group consisting of hydrogen.

In some embodiments, R¹ in the compound represented by formula I or the salt thereof is selected from the group consisting of:

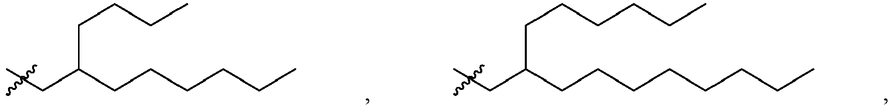

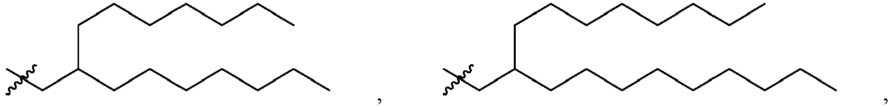

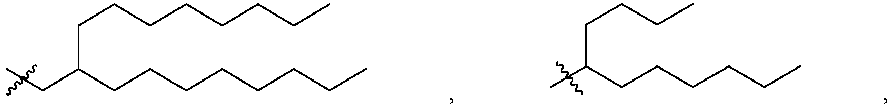

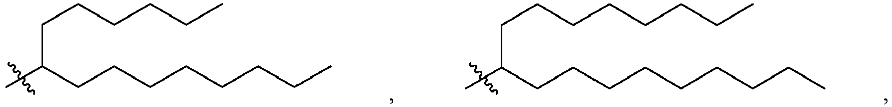

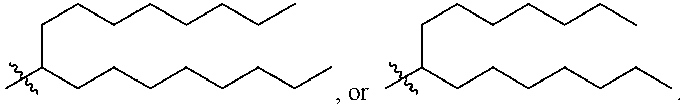

Typical compounds represented by formula I or pharmaceutically acceptable salts thereof include, but are not limited to:

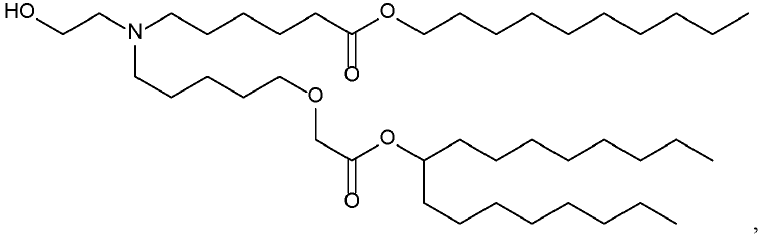

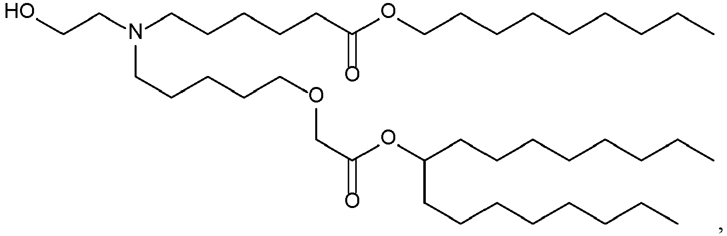

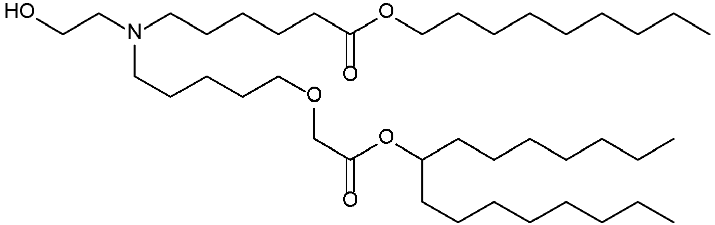

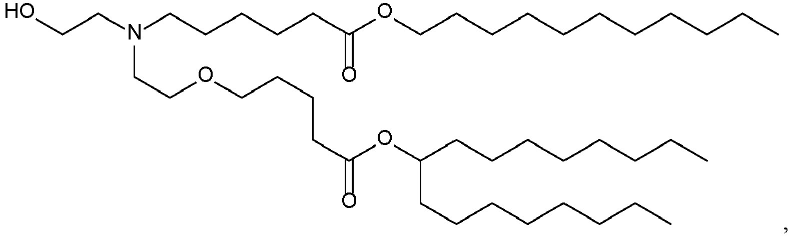

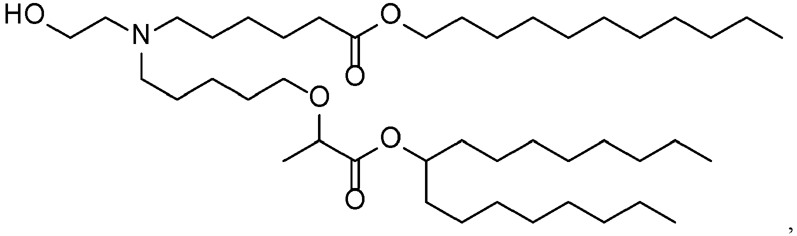

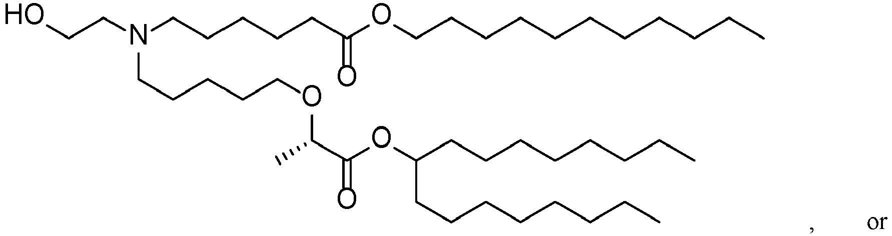

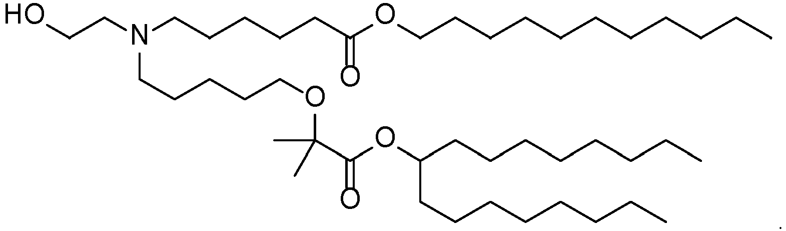

The disclosure also provides an isotopically substituted form of the aforementioned compound or the salt thereof; preferably, the isotopically substituted form is a form substituted with a deuterium atom.

The disclosure also provides a lipid particle comprising the aforementioned compound or the salt thereof. Further, in some embodiments, the lipid particle further comprises an active agent.

In some embodiments, the active agent is selected from the group consisting of a polynucleotide or a nucleic acid (e.g., a ribonucleic acid or a deoxyribonucleic acid).

In some embodiments, the active agent is selected from the group consisting of an mRNA. The disclosure also provides a pharmaceutical composition comprising the aforementioned lipid particle and a pharmaceutically acceptable excipient. In certain embodiments, the pharmaceutical composition comprises 0.01%-99.99% of the pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The disclosure also provides use of the aforementioned compound or the salt thereof, or the isotopically substituted form, or the aforementioned lipid particle, or the aforementioned pharmaceutical composition, in the preparation of a medicament for preventing and/or treating a disease or disorder that induces an immune response in a subj ect.

The disclosure also provides use of the aforementioned compound or the salt thereof, or the isotopically substituted form, or the aforementioned lipid particle, or the aforementioned pharmaceutical composition, in the preparation of a medicament for preventing and/or treating a disease or disorder associated with polypeptide overexpression.

The disclosure also provides use of the aforementioned compound or the salt thereof, or the isotopically substituted form, or the aforementioned lipid particle, or the aforementioned pharmaceutical composition, in the preparation of a medicament for preventing and/or treating a disease or disorder associated with insufficient polypeptide expression.

In some embodiments, the disease or disorder includes, but is not limited to: cancer, infection, autoimmune disease, neurodegenerative disease, and inflammation.

The disclosure also provides a method for preventing and/or treating a disease or disorder that induces an immune response in a subject, comprising administering to the patient the aforementioned compound or the salt thereof, or the aforementioned lipid particle, or the aforementioned pharmaceutical composition.

The disclosure also provides a method for preventing and/or treating a disease or disorder associated with polypeptide overexpression, comprising administering to the patient the aforementioned compound or the salt thereof, or the aforementioned lipid particle, or the aforementioned pharmaceutical composition.

The disclosure also provides a method for preventing and/or treating a disease or disorder associated with insufficient polypeptide expression, comprising administering to the patient the aforementioned compound or the salt thereof, or the aforementioned lipid particle, or the aforementioned pharmaceutical composition.

In another aspect, the disclosure also provides the aforementioned compound or the salt thereof, or the aforementioned lipid particle, or the aforementioned pharmaceutical composition, for use in preventing and/or treating a disease or disorder that induces an immune response in a subject.

The disclosure also provides the aforementioned compound or the salt thereof, or the aforementioned lipid particle, or the aforementioned pharmaceutical composition, for use in preventing and/or treating a disease or disorder associated with polypeptide overexpression.

The disclosure also provides the aforementioned compound or the salt thereof, or the aforementioned lipid particle, or the aforementioned pharmaceutical composition, for use in preventing and/or treating a disease or disorder associated with insufficient polypeptide expression.

The disclosure also provides use of the aforementioned compound or the salt thereof, or the isotopically substituted form, or the aforementioned lipid particle, or the aforementioned pharmaceutical composition, in the preparation of a medicament for preventing and/or treating cancer, infection, autoimmune disease, neurodegenerative disease, and inflammation.

The disclosure also provides a method for preventing and/or treating cancer, infection, autoimmune disease, neurodegenerative disease, and inflammation, comprising administering to the patient the aforementioned compound or the salt thereof, or the aforementioned lipid particle, or the aforementioned pharmaceutical composition.

The compound salt described in the disclosure includes "acid" addition salts and "base" addition salts. For example, salts formed by acid-base reactions with basic groups (amino groups) are included, and the acid includes organic or inorganic acids. In addition, the compound salt also includes salts formed by quaternization with basic groups (amino groups); the quaternizing reagent includes straight-chain or branched-chain chlorinated hydrocarbons.

The compounds of the disclosure may exist in specific geometric or stereoisomeric forms. The disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the disclosure. Additional asymmetric carbon atoms can be present in substituents such as an alkyl group. All such isomers and mixtures thereof are included within the scope of the disclosure. The compounds of the disclosure containing asymmetric carbon atoms can be separated in optically active pure form or in racemic form. The optically active pure form can be isolated from a racemic mixture or synthesized using chiral starting materials or chiral reagents.

Optically active (R)- and (S)-enantiomers and D- and L-isomers can be prepared by chiral synthesis, chiral reagents or other conventional techniques. If one enantiomer of a certain compound of the disclosure is desired, it can be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines). In the chemical structures of the compounds of the disclosure, a bond " 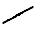 " represents an unspecified configuration; that is, if chiral isomers exist in the chemical structures, the bond " 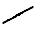 " may be " 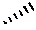 " or " 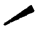 ", or both the configurations of " 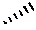 " and " 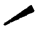 " are included simultaneously. In the chemical structures of the compounds of the disclosure, a bond " 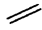 " is not specified with a configuration; that is, they may be in a Z configuration or an E configuration, or contain both configurations.

The compounds and intermediates of the disclosure may also exist in different tautomeric forms, and all such forms are included within the scope of the disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

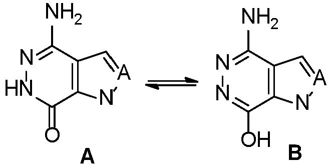

All compounds in the disclosure can be drawn as form A or form B. All tautomeric forms are within the scope of the disclosure. The names of the compounds do not exclude any tautomers.

The disclosure also includes isotopically labeled compounds that are identical to those recited herein but have one or more atoms replaced by an atom having an atomic mass or mass number different from the atomic mass or mass number usually found in nature. Examples of isotopes that can be incorporated into the compounds of the disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The disclosure also includes various deuterated forms of the compound of formula (I). Each available hydrogen atom connected to a carbon atom may be independently replaced by a deuterium atom. Those skilled in the art are able to synthesize the deuterated forms of the compound of formula (I) according to the relevant literature. Commercially available deuterated starting materials can be used in preparing the deuterated forms of the compound of formula (I), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

"Optionally" or "optional" means that the event or circumstance subsequently described may, but does not necessarily, occur, and this description includes instances where the event or circumstance occurs or does not occur. For example, "C₁₋₆ alkyl that is optionally substituted with a halogen or cyano" means that the halogen or cyano may, but does not necessarily, exist, and this description includes the instance where the alkyl is substituted with a halogen or cyano and the instance where the alkyl is not substituted with a halogen or cyano.

### Terms and definitions:

"Pharmaceutical composition" refers to a mixture containing one or more of the compounds or the physiologically/pharmaceutically acceptable salts or pro-drugs thereof described herein, and other chemical components, as well as other components, e.g., physiologically/pharmaceutically acceptable carriers and excipients. The pharmaceutical composition is intended to promote administration to an organism and facilitate the absorption of the active ingredient so that it can exert its biological activity. "Pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent or emulsifier that has been approved by the U.S. Food and Drug Administration as acceptable for use in humans or livestock animals.

"Effective amount" or "therapeutically effective amount" described in the disclosure includes an amount sufficient to ameliorate or prevent a symptom or disorder of a medical disorder. An effective amount also refers to an amount sufficient to allow or facilitate diagnosis. The effective amount for a particular patient or veterinary subject may vary depending on factors such as the disorder to be treated, the general health of the patient, the method and route and dosage of administration, and the severity of side effects. An effective amount may be the maximum dose or administration regimen to avoid significant side effects or toxic effects.

The term "nucleic acid" is a polymer composed of nucleotides, such as deoxyribonucleotides (DNA) or ribonucleotides (RNA).

The term "oligonucleotide" describes a single- or double-stranded nucleotide polymer that is 2 to 100 nucleotides in length. "Polynucleotide" refers to a single- or double-stranded polymer composed of nucleotide monomers. In some embodiments, the polynucleotide consists of 100 or more nucleotides.

In some other embodiments, exemplary polynucleotides include, but are not limited to, deoxyribonucleotides (DNA), ribonucleic acids (RNA, including messenger RNA), RNAi-inducing agents, shRNA, siRNA, miRNA, antisense RNA, and analogs.

The terms "polypeptide", "peptide", and "protein" are used interchangeably herein to refer to a polymer of amino acid residues. The terms apply to amino acid polymers in which one or more amino acid residues are artificial chemical analogs of corresponding naturally occurring amino acids, as well as to naturally occurring amino acid polymers. The term "white blood cells" relates to white blood cells (WBCs) produced and derived from pluripotent hematopoietic stem cells in the bone marrow. White blood cells have nuclei and can be classified into five main types, including, neutrophils, eosinophils, basophils, lymphocytes, and monocytes, based on functional or physical characteristics. "Alkyl" refers to saturated aliphatic hydrocarbon groups, including straight-chain and branched-chain alkyl groups. In some embodiments, alkyl groups have 1-4 carbon atoms and are also known as C₁₋₄ alkyl. In some embodiments, alkyl groups have 10-22 carbon atoms and are also known as C₁₀₋₂₂ alkyl. In some embodiments, alkyl groups have 4-22 carbon atoms and are also known as C₄₋₂₂ alkyl.

Alkyl groups may be unsubstituted or substituted with one or more groups selected from the group consisting of halogen, hydroxy, amino, oxo, alkyl, alkenyl, alkoxycarbonyl, amido, alkylamido, dialkylamido, nitro, amino, alkylamino, dialkylamino, carboxyl, thio, and thioalkyl.

"Heteroalkyl" refers to a straight-chain or branched-chain alkyl group that preferably has 1 to 14 carbon atoms and more preferably has 2 to 10 carbon atoms in the chain, wherein one or more of the carbon atoms are substituted with heteroatoms selected from the group consisting of S, O, and N. Exemplary heteroalkyl groups include alkyl ethers, secondary and tertiary alkyl amines, amides, alkyl sulfides, and the like.

Heteroalkyl groups may be unsubstituted or substituted with one or more groups selected from the group consisting of halogen, hydroxy, alkyl, amino, oxo, alkenyl, alkoxycarbonyl, amido, alkylamido, dialkylamido, nitro, amino, alkylamino, dialkylamino, carboxyl, thio, and thioalkyl.

"Alkenyl" refers to unsaturated aliphatic hydrocarbon groups, including straight-chain and branched-chain alkenyl groups. In some embodiments, alkenyl groups have 1-4 carbon atoms and are also known as C₁₋₄ alkenyl. In some embodiments, alkenyl groups have 10-22 carbon atoms and are also known as C₁₀₋₂₂ alkenyl. In some embodiments, alkenyl groups have 4-22 carbon atoms and are also known as C₄₋₂₂ alkenyl. Exemplary alkenyl groups include ethenyl, propenyl, n-butenyl, isobutenyl, 3-methylbut-2-enyl, n-pentenyl, heptenyl, octenyl, cyclohexyl-butenyl, and decenyl.

Alkenyl groups may be unsubstituted or substituted with one or more groups selected from the group consisting of halogen, hydroxy, amino, oxo, alkyl, alkenyl, alkoxycarbonyl, amido, alkylamido, dialkylamido, nitro, amino, alkylamino, dialkylamino, carboxyl, thio, and thioalkyl.

"Monovalent group" refers to a group formed by "formally" eliminating a monovalent atom or group from a compound. "-ylene" refers to a group formed by "formally" eliminating two monovalent atoms or atomic groups or one divalent atom or atomic group from a compound.

The term "alkylene" refers to the remainder of an alkane molecule after 2 hydrogen atoms are removed; in some embodiments, alkylene groups have 1-4 carbon atoms and are also known as C₁₋₄ alkylene. In some embodiments, alkyl groups have 10-22 carbon atoms and are also known as C₁₀₋₂₂ alkylene. In some embodiments, alkyl groups have 4-22 carbon atoms and are also known as C₄₋₂₂ alkylene.

Likewise, the definitions of "alkyleneoxy", "alkenylene", "alkenyleneoxy", "cycloalkylene", and "heterocycloalkylene" are similar to that of "alkylene".

The term "hydroxy" refers to the -OH group.

The term "halogen" refers to fluorine, chlorine, bromine, or iodine.

The term "cyano" refers to -NH₂.

The term "oxo" refers to the =O substituent.

"Substituted" means that one or more, preferably up to 5, and more preferably 1 to 3, hydrogen atoms in the group are independently substituted with a corresponding number of substituents. It goes without saying that a substituent is only in its possible chemical position, and those skilled in the art will be able to determine (experimentally or theoretically) possible or impossible substitutions without undue effort.

### BRIEF DESCRIPTION OF THE DRAWINGS

FIG. 1: the protein expression levels in mice intramuscularly injected with hepatocyte growth factor mRNA lipid nanoparticles.
FIG. 2: the measurements of fluorescence intensity in mice injected with luciferase mRNA lipid nanoparticles via the tail veins.
FIG. 3: the cationic lipid concentrations in the livers of mice injected with mRNA lipid nanoparticles via the tail veins.
FIG. 4: the cationic lipid concentrations in the spleens of mice injected with mRNA lipid nanoparticles via the tail veins.
FIG. 5: the cationic lipid concentrations in the plasma of mice injected with mRNA lipid nanoparticles via the tail veins.
FIG. 6: the measurements of the interleukin-6 (IL-6) concentration in the serum of mice injected with mRNA lipid nanoparticles via the tail veins.

### DETAILED DESCRIPTION

The disclosure is further described below using examples. However, these examples do not limit the scope of the disclosure.

Experimental methods without conditions specified in the examples of the disclosure were generally conducted under conventional conditions, or under conditions recommended by the manufacturers of the starting materials or commercial products. Reagents without specific origins indicated are commercially available conventional reagents.

The structures of the compounds were determined by nuclear magnetic resonance (NMR) spectroscopy or/and mass spectrometry (MS). The NMR shifts (δ) are given in 10⁻⁶ (ppm). The NMR analyses were performed on a Bruker AVANCE-400 nuclear magnetic resonance instrument, with dimethyl sulfoxide-D6 (DMSO-*d₆*), chloroform-D (CDCl₃), and methanol-D4 (methanol-*d₄*) as solvents and tetramethylsilane (TMS) as an internal standard.

The HPLC analyses were performed using an Agilent1100 high pressure liquid chromatograph, a GAS15B DAD ultraviolet detector, and a Water Vbridge C18 150 × 4.6 mm 5 µm chromatography column.

The MS analyses were performed using an Agilent6120 triple quadrupole mass spectrometer, a G1315D DAD detector, and a Waters Xbridge C18 4.6 × 50 mm, 5 µm chromatography column, with scanning performed in positive/negative ion mode with a mass scan range of 80-1200.

The thin-layer chromatography silica gel plates used were Yantai Huanghai HSGF254 silica gel plates. The silica gel plates used in the thin-layer chromatography (TLC) analyses had a layer thickness of 0.2 mm ± 0.03 mm, and those used in the thin-layer chromatography separation and purification had a layer thickness of 0.4 mm-0.5 mm. The flash column purification system used was Combiflash Rf150 (TELEDYNE ISCO) or Isolara one (Biotage).

In the normal-phase column chromatography steps, a 200-300 mesh or 300-400 mesh Yantai Huanghai silica gel was generally used as the carrier, or a Changzhou Santai pre-fill ultrapure normal-phase silica gel column (40-63 µm, 60 g, 24 g, 40 g, 120 g, or other specifications) was used.

The known starting materials in the disclosure may be synthesized by using or following methods known in the art, or may be purchased from Shanghai Titan Scientific, ABCR GmbH & Co. KG, Acros Organics, Aldrich Chemical Company, Accela ChemBio Inc., and Bide Pharmatech, among others.

In the examples, the reactions can all be performed in a nitrogen atmosphere unless otherwise specified.

The nitrogen atmosphere means that the reaction flask is connected to a balloon containing about 1 L of nitrogen gas.

The hydrogen atmosphere means that the reaction flask is connected to a balloon containing about 1 L of hydrogen gas.

The hydrogen gas was prepared by a QPH-1L hydrogen generator from Shanghai Quan Pu Scientific Instruments Inc.

The nitrogen atmosphere or the hydrogenation atmosphere was generally formed by 3 cycles of vacuumization and nitrogen or hydrogen filling.

In the examples, the solutions refer to aqueous solutions unless otherwise specified.

In the examples, the reaction temperature was room temperature, i.e., 20 °C-30 °C, unless otherwise specified.

The monitoring of reaction progress in the examples was performed using thin-layer chromatography (TLC). For the developing solvents used in the reactions, the eluent systems used in the column chromatography purification, and the developing solvent systems used in the thin-layer chromatography analyses, the volume ratio of the solvents was adjusted depending on the polarity of the compound, or by adding a small amount of basic or acidic reagents such as triethylamine and acetic acid.

### Example 1

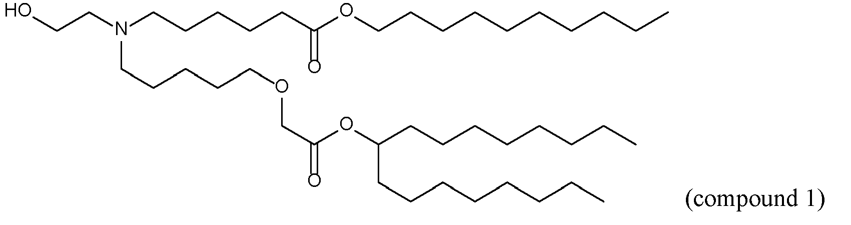

### Step 1)

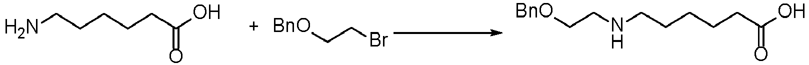

6-Aminocaproic acid (21.5 g, 100 mmol) was dissolved in a mixed solution of ethanol and water (EtOH/H₂O, v/v = 2:1, 200 mL). CsOH·H₂O (4.0 g, 100 mmol) was added, and benzyloxybromoethane (13.1 g, 100 mmol) was added. The mixture was left to react at room temperature for 40 h. The reaction mixture was concentrated to remove most of the ethanol, and the residue was directly purified by reversed-phase column chromatography (acetonitrile/water/0.1% trifluoroacetic acid). The eluate was directly concentrated to dryness under reduced pressure to give a white solid (5.6 g, yield: 21%). MS: 266.2[M+H]⁺.

¹H NMR (400 MHz, D₂O) δ 7.36 (s, 5H), 4.53 (s, 2H), 3.72 - 3.67 (m, 2H), 3.20 - 3.14 (m, 2H), 2.95 - 2.88 (m, 2H), 2.10 (t, *J* = 7.3 Hz, 2H), 1.57 (dd, *J* = 15.3, 7.7 Hz, 2H), 1.47 (dd, *J* = 15.0, 7.5 Hz, 2H), 1.30 - 1.22 (m, 2H).

### Step 2)

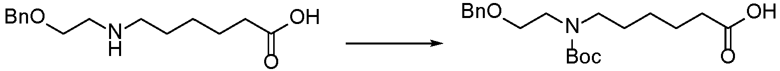

6-((2-(Benzyloxy)ethyl)amino)hexanoic acid (5.6 g, 21.1 mmol) was weighed into a reaction flask. 1,4-Dioxane (80 mL) and an aqueous sodium hydroxide solution (42.2 mL, 1.0 M) were added, and Boc₂O (5.5 g, 25.2 mmol) was added dropwise. The mixture was left to react at room temperature for 1 h, and LC-MS analysis showed that the reaction was complete. The reaction mixture was concentrated to remove the dioxane. The pH of the aqueous phase was adjusted to about 5 with dilute hydrochloric acid, and extraction was performed with ethyl acetate (50 mL × 3). The organic phases were combined, dried, and concentrated to give a nearly colorless gum (7.2 g, yield: 93%). The product was directly used in the next step.

MS: 366.2[M+H]⁺.

### Step 3)

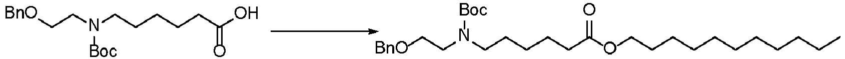

6-((2-(Benzyloxy)ethyl)(tert-butoxycarbonyl)amino)hexanoic acid (6.1 g, 16.7 mmol) was weighed into a reaction flask, and dichloromethane (150 mL) was added. Under an ice bath, undecanol (2.73 g, 15.9 mmol), DMAP (408 mg, 3.34 mmol), and DIPEA (4.3 g, 33.4 mmol) were sequentially added, and EDCI (3.84 g, 20.0 mmol) was added. After 10 minutes of stirring, the ice bath was removed. The mixture was left to react overnight at room temperature, and TLC monitoring showed that the reaction was complete. Water (100 mL) was added to the reaction mixture. The organic phase was separated, washed with dilute hydrochloric acid, water, and sodium bicarbonate in sequence, dried, concentrated, and purified by column chromatography (PE:EtOAc = 20:1-10:1) to give a nearly colorless gum (6.8 g, yield: 82%).

MS: 520.4[M+H]⁺.

¹H NMR (400 MHz, CDCl₃) δ 7.30 - 7.20 (m, 5H), 4.44 (s, 2H), 3.98 (t, *J* = 6.7 Hz, 2H), 3.51 (d, *J* = 18.6 Hz, 2H), 3.32 (d, *J* = 21.5 Hz, 2H), 3.16 (s, 2H), 2.21 (t, *J* = 7.5 Hz, 2H), 1.55 (tt, *J* = 13.3, 6.8 Hz, 6H), 1.36 (d, *J* = 8.4 Hz, 9H), 1.21 (d, *J* = 15.5 Hz, 18H), 0.81 (t, *J* = 6.8 Hz, 3H).

### Step 4)

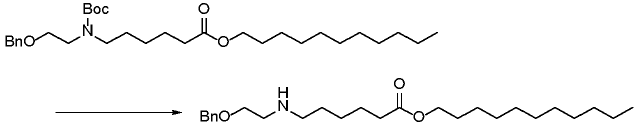

Undecyl 6-((2-(benzyloxy)ethyl)(tert-butoxycarbonyl)amino)hexanoate (7.6 g, 14.6 mmol) was weighed out and dissolved in dichloromethane, and TFA (20 mL) was added. The mixture was left to react at room temperature for 2 h, and the reaction mixture was directly concentrated. The residue was dissolved in dichloromethane (100 mL), and saturated sodium bicarbonate (100 mL) was added. The organic phase was separated, dried, and concentrated to give a gum. The gum was then dissolved in dichloromethane, and HCl/dioxane (4.0 M, 4 mL) was added. The mixture was directly concentrated to give a white solid (5.0 g, yield: 81%).

MS: 420.3 [M+H]⁺.

¹H NMR (400 MHz, CDCl₃) δ 9.51 (s, 2H), 7.39 - 7.28 (m, 5H), 4.57 (s, 2H), 4.03 (t, *J* = 6.8 Hz, 2H), 3.88 (t, *J* = 4.8 Hz, 2H), 3.18 (s, 2H), 3.03 (d, *J* = 3.4 Hz, 2H), 2.28 (t, *J* = 7.4 Hz, 2H), 2.02 (s, 1H), 1.93 - 1.84 (m, 2H), 1.61 (dd, *J* = 13.8, 6.7 Hz, 4H), 1.42 - 1. 17 (m, 18H), 0.88 (t, *J* = 6.8 Hz, 3H).

### Step 5)

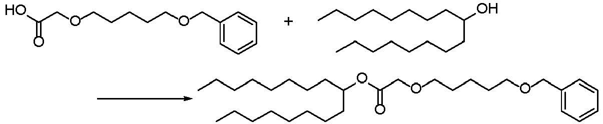

2-((5-(Benzyloxy)pentyl)oxy)acetic acid (prepared with reference to Chemical and Pharmaceutical Bulletin, 1992, vol. 40, #3, p. 617-623) (7.6 g, 30.12 mmol) was weighed into a reaction flask, and dichloromethane (150 mL) was added. Under an ice bath, heptadecan-9-ol (prepared with reference to WO2020/219876) (6.18 g, 24.1 mmol) and DMAP (3.68 g, 30.12 mmol) were sequentially added, and EDCI (6.93 g, 36.14 mmol) was added. After 10 minutes of stirring, the ice bath was removed. The mixture was left to react overnight at room temperature, and TLC monitoring showed that the reaction was complete. Water (100 mL) was added to the reaction mixture. The organic phase was separated, washed with dilute hydrochloric acid, water, and sodium bicarbonate in sequence, dried, concentrated, and purified by column chromatography (PE:EtOAc = 50:1-10:1) to give a nearly colorless gum (10.9 g, yield: 92%).

¹H NMR (400 MHz, CDCl₃) δ 7.36 - 7.31 (m, 4H), 7.30 - 7.26 (m, 1H), 4.96 (p, *J* = 6.3 Hz, 1H), 4.50 (s, 2H), 4.04 (s, 2H), 3.52 (t, *J* = 6.6 Hz, 2H), 3.47 (t, *J* = 6.6 Hz, 2H), 1.69 - 1.61 (m, 4H), 1.56 (d, *J* = 16.5 Hz, 6H), 1.25 (s, 24H), 0.88 (t, *J* = 6.8 Hz, 6H).

### Step 6)

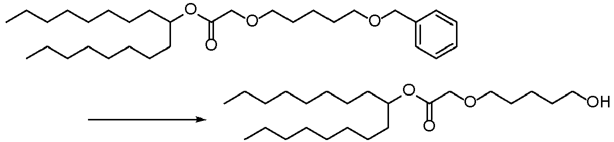

Heptadecan-9-yl 2-((5-(benzyloxy)pentyl)oxy)acetate (4.0 g, 8.15 mmol) was weighed out and dissolved in THF (100 mL). Pd(OH)₂/C (400 mg, 20%) was added, and hydrogen gas was introduced into the system. The mixture was left to react at room temperature for 2 h, and TLC monitoring showed the reaction was complete. The reaction mixture was filtered, and the filtrate was concentrated to give a colorless gum (3.2 g, yield: 98%). The product was directly used in the next step.

¹H NMR (400 MHz, CDCl₃) δ 4.97 (dd, *J* = 12.4, 6.1 Hz, 1H), 4.04 (s, 2H), 3.66 (t, *J* = 6.5 Hz, 2H), 3.54 (t, *J* =6.5 Hz, 2H), 1.71 - 1.57 (m, 4H), 1.56 - 1.42 (m, 6H), 1.25 (s, 24H), 0.88 (t, *J* = 6.8 Hz, 6H).

### Step 7)

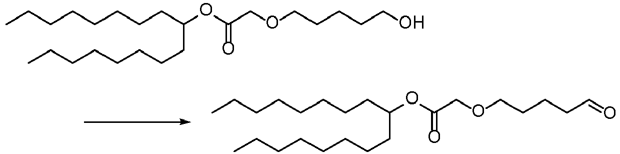

Heptadecan-9-yl 2-((5-hydroxypentyl)oxy)acetate (2.4 g, 6.0 mmol) was weighed out and dissolved in dichloromethane (100 mL). Under an ice bath, DMP (3.82 g, 9.0 mmol) was added, and the mixture was left to react at room temperature for 1.5 h. A saturated aqueous sodium thiosulfate solution (100 mL) was added, and the mixture was stirred for 30 minutes. The organic phase was separated, washed with a saturated aqueous sodium bicarbonate solution (100 mL × 2), dried, and concentrated to give a light yellow gum (2.5 g). The product was directly used in the next step.

¹H NMR (400 MHz, CDCl₃) δ 9.78 (s, 1H), 4.96 (p, *J* = 6.3 Hz, 1H), 4.04 (s, 2H), 3.55 (t, *J* = 6.1 Hz, 2H), 2.49 (td, *J* = 7.2, 1.5 Hz, 2H), 1.81 - 1.72 (m, 2H), 1.68 (d, *J* = 7.6 Hz, 2H), 1.60 - 1.48 (m, 4H), 1.26 (s, 24H), 0.88 (t, *J* = 6.8 Hz, 6H).

### Step 8)

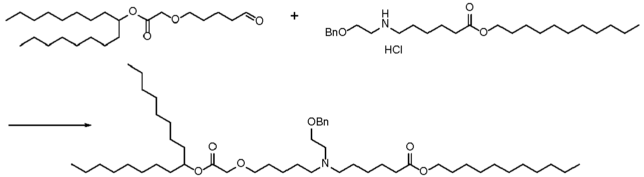

Heptadecan-9-yl 2-((5-oxopentyl)oxy)acetate (2.4 g, 6 mmol) was weighed out and dissolved in dichloromethane (50 mL). Undecyl 6-((2-(benzyloxy)ethyl)amino)hexanoate hydrochloride (2.46 g, 5.4 mmol) was added, and DIPEA (1.16 g, 9.0 mmol) and acetic acid (1.08 g, 18.0 mmol) were added. The mixture was stirred until complete dissolution was achieved. Under an ice bath, NaBH(OAc)₃ (3.18 g, 15.0 mmol) was added. The mixture was left to react overnight at room temperature, and TLC monitoring showed that the reaction was complete. Water (100 mL) was added. The organic phase was separated, washed with a saturated aqueous sodium bicarbonate solution (50 mL × 1), dried, concentrated, and purified by column chromatography (PE:EtOAc = 5:1-2:1) to give a colorless liquid (4.1 g, yield: 85%).

¹H NMR (400 MHz, CDCl₃) δ 7.33 (d, *J* = 4.5 Hz, 4H), 7.30 - 7.26 (m, 1H), 4.99 - 4.91 (m, 1H), 4.52 (s, 2H), 4.08 - 4.02 (m, 4H), 3.57 - 3.47 (m, 4H), 2.68 (t, *J* = 5.8 Hz, 2H), 2.45 (s, 4H), 2.28 (t, *J* = 7.5 Hz, 2H), 1.67 - 1.57 (m, 6H), 1.48 (dd, *J* = 37.5, 6.0 Hz, 6H), 1.38 - 1.18 (m, 46H), 0.88 (t, *J* = 6.8 Hz, 9H).

### Step 9)

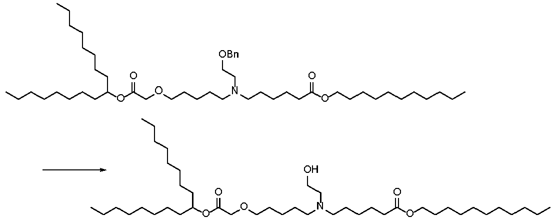

Undecyl 6-((2-(benzyloxy)ethyl)(5-(2-(heptadecan-9-yloxy)-2-oxoethoxy)pentyl)amino)hexanoat e (1 g, 1.25 mmol) was weighed into a reaction flask and dissolved in ethanol. Pd(OH)₂/C(500 mg, 20%) was added, and hydrogen gas was introduced into the system. The mixture was left to react overnight at room temperature, and TLC monitoring showed that the reaction was complete. The reaction mixture was filtered, and the filtrate was concentrated to give a colorless gum. Column chromatography purification was performed (CH₂Cl₂:MeOH = 10:1) to give a colorless gum (450 mg, yield: 51%).

¹H NMR (400 MHz, CDCl₃) δ 5.00 - 4.89 (m, 1H), 4.09 - 4.01 (m, 4H), 3.61 (s, 2H), 3.52 (t, *J* = 6.4 Hz, 2H), 2.68 (s, 2H), 2.57 (s, 4H), 2.30 (t, *J* = 7.4 Hz, 2H), 1.70 - 1.59 (m, 6H), 1.53 (s, 8H), 1.43 - 1.18 (m, 44H), 0.88 (t, *J* = 6.7 Hz, 9H).

### Example 2

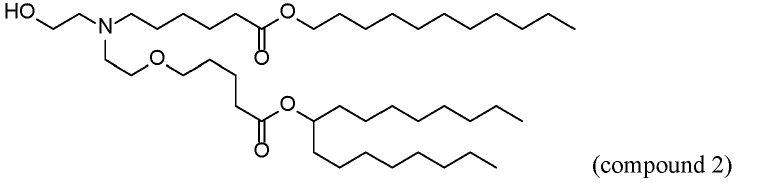

### Step 1)

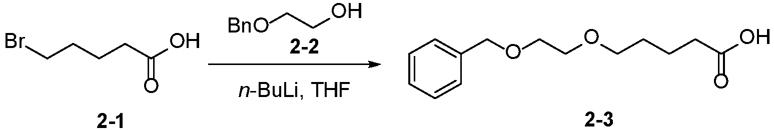

Compound **2-2** (50.4 g, 331 mmol) was weighed out and dissolved in THF (400 mL), and *n*-BuLi (206 mL, 1.6 M) was slowly added dropwise under a dry ice ethanol bath. The mixture was stirred at the same temperature for 1 hour. Compound **2-1** (20.0 g, 110 mmol) was dissolved in THF (100 mL), and the solution was slowly added dropwise to the reaction system under a dry ice ethanol bath. The mixture was stirred at the same temperature for 1 hour and then stirred overnight at room temperature. A sample was taken and analyzed by LC-MS, and the results showed that the starting materials were completely consumed. 1 L of water was added, and extraction was performed with ethyl acetate (1 L × 3). The organic phase was washed with saturated brine, then dried over anhydrous sodium sulfate, and filtered. The filtrate was concentrated under reduced pressure and purified by normal-phase column chromatography (PE:EA = 5:1) to give a yellow oil (9.00 g, yield: 32.3%).

MS: 253.1[M+H]⁺.

¹H NMR (400 MHz, CDCl₃) *δ* 7.40-7.24 (m, 5H), 4.57 (s, 2H), 3.65-3.57 (m, 4H), 3.49 (t, *J* = 6.0 Hz, 2H), 2.39 (t, *J* = 7.2 Hz, 2H), 1.77-1.61 (m, 4H).

### Step 2)

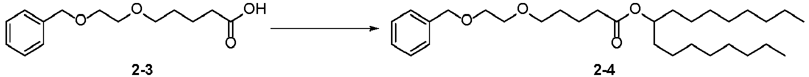

Compound **2-3** (1.00 g, 3.96 mmol) was dissolved in DCM (20 mL), and heptan-9-ol (914 mg, 3.56 mmol), DMAP (96.0 mg, 786 µmol), DIEA (2.04 g, 15.8 mmol), and EDCI (1.14 g, 9.95 mmol) were sequentially added under an ice-water bath. The mixture was stirred overnight at 40 °C. Samples were taken and analyzed by LCMS and TLC (PE:EA = 10:1), and the results showed that the starting material was completely consumed. 100 mL of water was added, and extraction was performed with ethyl acetate (100 mL × 3). The organic phase was washed with saturated brine, then dried over anhydrous sodium sulfate, and filtered. The filtrate was concentrated under reduced pressure and purified by normal-phase column chromatography (PE:EA = 20:1) to give a colorless oil (1.50 g, yield: 77.1%).

MS: 5 13.3 [M+Nat.

¹H NMR (400 MHz, CDCl₃) *δ* 7.38-7.24 (m, 5H), 4.90-4.82 (m, 1H), 4.57 (s, 2H), 3.64-3.58 (m, 4H), 3.48 (t, *J* = 6.4 Hz, 2H), 2.31 (t, *J* = 7.2 Hz, 2H), 1.75-1.59 (m, 4H), 1.55-1.45 (m, 4H), 1.34-1.19 (m, 24H), 0.87 (t, *J* = 6.8 Hz, 6H).

### Step 3)

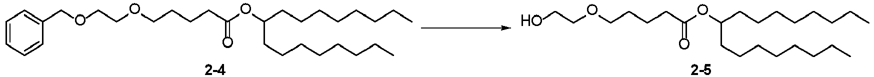

Compound **2-4** (1.50 g, 3.06 mmol) was weighed out and dissolved in THF (30 mL), and Pd(OH)₂/C (300 mg, 20wt%) was added. The system was purged with hydrogen gas, and the mixture was stirred overnight at room temperature. A sample was taken and analyzed by TLC (PE:EA = 20:1), and the results showed that the starting material was completely consumed. The reaction mixture was filtered, and the filtrate was concentrated and purified by normal-phase column chromatography (PE:EA = 20:1) to give a colorless oil (1.10 g, yield: 89.8%).

MS: 423.3[M+Na]⁺.

¹H NMR (400 MHz, CDCl₃) *δ* 4.91-4.82 (m, 1H), 3.76-3.69 (m, 2H), 3.55-3.46 (m, 4H), 2.32 (t, *J* = 7.2 Hz, 2H), 1.95 (s, 1H), 1.76-1.58 (m, 4H), 1.57-1.44 (m, 4H), 1.36-1.18 (m, 24H), 0.87 (t, *J* = 6.8 Hz, 6H).

### Step 4)

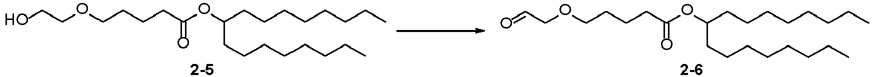

Compound **2-5** (120 mg, 300 µmol) was dissolved in DCM (4 mL), and DMP (190 mg, 448 µmol) was added under an ice-water bath. The mixture was stirred at room temperature for 2 h. A sample was taken and analyzed by TLC (PE:EA = 5:1), and the results showed that the starting material was completely consumed. 1 mL of a saturated aqueous sodium thiosulfate solution and 20 mL of a saturated aqueous sodium bicarbonate solution were added to the reaction mixture, and extraction was performed with ethyl acetate (20 mL × 3). The organic phase was washed with saturated brine, then dried over anhydrous sodium sulfate, and filtered. The filtrate was concentrated under reduced pressure to give a light yellow oil (80 mg). The product was directly used in the next step without purification.

¹H NMR (400 MHz, CDCl₃) *δ* 9.73 (s, 1H), 4.91-4.81 (m, 1H), 4.09 (s, 1H), 4.06 (s, 1H), 3.65-3.44 (m, 2H), 2.33 (t, *J* = 7.2 Hz, 2H), 1.79-1.59 (m, 4H), 1.57-1.43 (m, 4H), 1.36-1.16 (m, 24H), 0.87 (t, *J* = 6.8 Hz, 6H).

### Step 5)

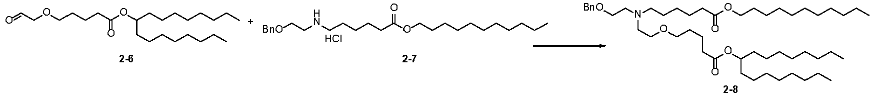

Compound **2-6** (1.09 g, 2.73 mmol) was dissolved in DCM (20 mL), and **2-7** (1.12 g, 2.46 mmol), DIEA (530 mg, 4.10 mmol), and AcOH (492 mg, 8.19 mmol) were sequentially added. After 10 min of stirring, NaBH(OAc)₃ (1.45 g, 6.84 mmol) was added, and the mixture was stirred overnight at room temperature. A sample was taken and analyzed by TLC (pure EA), and the results showed that the starting materials were completely consumed. 100 mL of a saturated aqueous sodium bicarbonate solution was added to the reaction mixture, and extraction was performed with ethyl acetate (100 mL × 3). The organic phase was washed with saturated brine, then dried over anhydrous sodium sulfate, and filtered. The filtrate was concentrated under reduced pressure and purified by normal-phase column chromatography (PE:EA = 2:1) to give a light yellow oil (290 mg, yield: 13.2%).

MS: 802.4[M+H]⁺.

¹H NMR (400 MHz, CDCl₃) *δ* 7.39-7.26 (m, 5H), 4.89-4.82 (m, 1H), 4.51 (s, 2H), 4.05 (t, *J* = 6.8 Hz, 2H), 3.67-3.44 (m, 4H), 3.41 (t, *J* = 6.4 Hz, 2H), 2.87-2.37 (m, 6H), 2.33-2.24 (m, 4H), 1.79-1.55 (m, 8H), 1.55-1.43 (m, 6H), 1.38-1.15 (m, 42H), 0.87 (t, *J* = 6.8 Hz, 9H).

### Step 6)

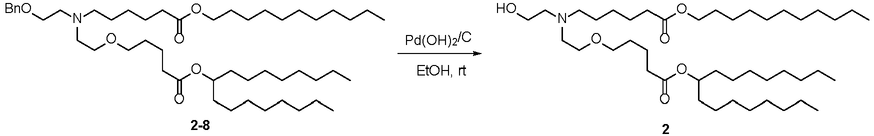

Compound **2-8** (390 mg, 486 µmol) was weighed out and dissolved in EtOH (15 mL), and Pd(OH)₂/C (390 mg, 20 wt%) was added. The system was purged with hydrogen gas, and the mixture was stirred overnight at room temperature. A sample was taken and analyzed by TLC (DCM:MeOH = 10:1), and the results showed that the starting material was completely consumed. The reaction mixture was filtered, and the filtrate was concentrated and purified by normal-phase column chromatography (DCM:MeOH = 20:1) to give a colorless oil (110 mg, yield: 31.8%).

MS: 712.2[M+H]⁺.

¹H NMR (400 MHz, CDCl₃) *δ* 4.89-4.81 (m, 1H), 4.05 (t, *J* = 6.8 Hz, 2H), 3.55 (t, *J* = 5.2 Hz, 2H), 3.48 (t, *J* = 5.6 Hz, 2H), 3.43 (t, *J* = 6.4 Hz, 2H), 2.71 (t, *J* = 5.6 Hz, 2H), 2.67 (t, *J* = 5.2 Hz, 2H), 2.59-2.52 (m, 2H), 2.34-2.26 (m, 4H), 1.72-1.56 (m, 8H), 1.53-1.44 (m, 6H), 1.36-1.21 (m, 42H), 0.89-0.85 (m, 9H).

### Example 3

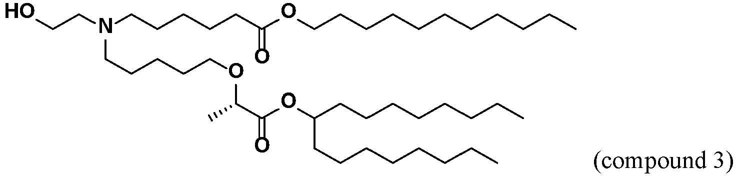

### Step 1)

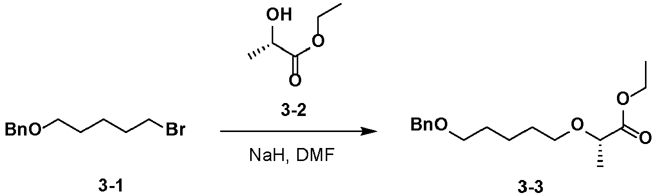

**3-2** (5.90 g, 50.0 mmol) was weighed out and dissolved in DMF (250 mL), and NaH (2.4 g, 60 mmol) was added portionwise under an ice-water bath. The mixture was stirred at the same temperature for 1 hour. **3-1** (12.9 g, 50.0 mmol) was added portionwise to the reaction system under an ice-water bath, and the mixture was stirred overnight at room temperature. 300 mL of water was added, and extraction was performed with ethyl acetate (300 mL × 2). The organic phase was washed with saturated brine, then dried over anhydrous sodium sulfate, and filtered. The filtrate was concentrated under reduced pressure and purified by normal-phase column chromatography (PE:EA = 10:1) to give a pale yellow oil (6.20 g, yield: 42.2%).

MS: 295.2[M+H].

¹H NMR (400 MHz, CDCl₃) *δ* 7.34-7.31 (m, 4H), 7.30-7.26 (m, 1H), 4.50 (s, 2H), 4.25-4.15 (m, 2H), 3.92 (q, *J* = 6.8 Hz, 1H), 3.59-3.53 (m, 1H), 3.47 (t, *J* = 6.4 Hz, 2H), 3.39-3.33 (m, 1H), 1.68-1.59 (m, 4H), 1.49-1.42 (m, 2H), 1.39 (d, *J* = 6.8 Hz, 3H), 1.28 (t, *J* = 6.4 Hz, 3H).

### Step 2)

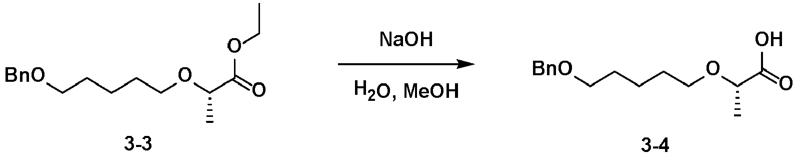

Compound **3-3** (7.40 g, 25.0 mmol) was dissolved in MeOH (150 mL), and a solution of NaOH (4.00 g, 100 mmol) in H₂O (40 mL) was slowly added under an ice-water bath. The mixture was stirred overnight at room temperature. The reaction mixture was cooled in an ice-water bath, and the pH was adjusted to about 3 with concentrated hydrochloric acid. Extraction was performed with DCM (200 mL × 3). The organic phase was washed with saturated brine, then dried over anhydrous sodium sulfate, and filtered. The filtrate was concentrated under reduced pressure to give a pale yellow oil (5.50 g, yield: 82.6%).

MS: 267.2[M+H].

¹H NMR (400 MHz, CDCl₃) *δ* 7.37-7.33 (m, 4H), 7.31-7.26 (m, 1H), 4.51 (s, 2H), 3.99 (q, *J=* 6.8 Hz, 1H), 3.60-3.40 (m, 4H), 1.69-1.61 (m, 4H), 1.50-1.44 (m, 5H).

### Step 3)

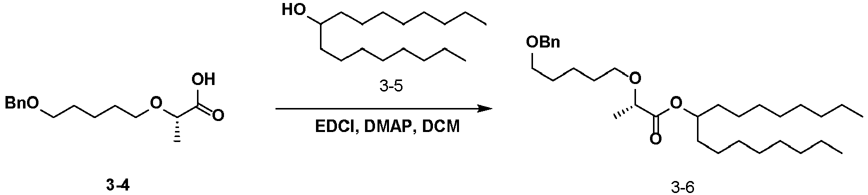

**3-4** (5.30 g, 20.0 mmol) and **3-5** (5.10 g, 20.0 mmol) were weighed out and dissolved in DCM (160 mL), and **DMAP** (4.90 g, 40.0 mmol) and **EDCI** (4.60 g, 24.0 mmol) were weighed out and sequentially added to the reaction mixture. The system was purged with hydrogen gas, and the mixture was stirred overnight at room temperature. The reaction mixture was purified by normal-phase column chromatography (PE:EA = 10:1) to give a pale yellow oil (8.60 g, 85.9%).

¹H NMR (400 MHz, CDCl₃) *δ* 7.34-7.31 (m, 4H), 7.31-7.27 (m, 1H), 4.96-4.91 (m, 1H), 4.50 (s, 2H), 3.91 (q, *J* = 6.8 Hz, 1H), 3.59-3.53 (m, 1H), 3.47 (t, *J* = 6.4 Hz, 2H), 3.38-3.32 (m, 1H), 1.68-1.60 (m, 4H), 1.54-1.52 (m, 4H), 1.49-1.43 (m, 2H), 1.39 (d, *J* = 6.8 Hz, 3H), 1.35-1.18 (m, 24H), 0.87 (t, *J* = 6.8 Hz, 6H).

### Step 4)

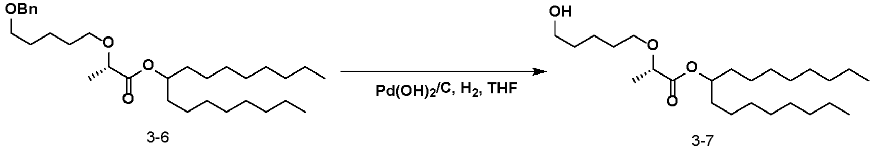

Compound **3-6** (5.00 g, 9.90 mmol) was dissolved in THF (100 mL), and Pd(OH)₂/C (500 mg) was added under a nitrogen atmosphere. The system was purged with hydrogen gas, and the mixture was stirred overnight at room temperature for hydrogenation. The Pd(OH)₂/C was removed by filtration and recovered. The filtrate was concentrated to dryness by rotary evaporation under reduced pressure and purified by normal-phase column chromatography (PE:EA = 5:1) to give a colorless oil (4.00 g, yield: 96.4%).

¹H NMR (400 MHz, CDCl₃) *δ* 4.96-4.92 (m, 1H), 3.91 (q, *J* = 6.8 Hz, 1H), 3.65 (t, *J* = 6.8 Hz, 2H), 3.59-3.53 (m, 1H), 3.40-3.36 (m, 1H), 1.68-1.56 (m, 4H), 1.54-1.52 (m, 4H), 1.49-1.41 (m, 2H), 1.39 (d, *J* = 6.8 Hz, 3H), 1.35-1.19 (m, 24H), 0.87 (t, *J* = 6.8 Hz, 6H).

### Step 5)

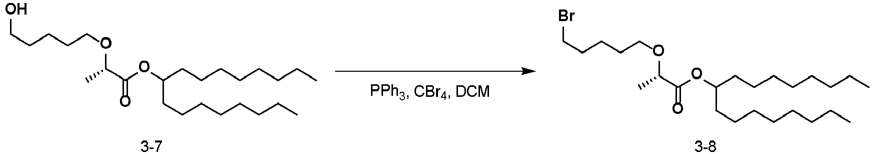

Compound 3-7 (4.10 g, 10.0 mmol) was dissolved in DCM (80 mL), and the solution was cooled in an ice-water bath. CBr₄ (5.00 g, 15 mmol) and PPh₃ (3.90 g, 15.0 mmol) were sequentially added under a nitrogen atmosphere. After 30 min of stirring at a constant temperature, the mixture was stirred overnight at room temperature. A sample was taken and analyzed by TLC (PE:EA = 5:1), and the results showed that the starting material was completely consumed. The reaction mixture was purified by normal-phase column chromatography (PE:EA = 10: 1) to give a light yellow oil (4.30 g, 90.0%).

¹H NMR (400 MHz, CDCl₃) *δ* 4.97-4.90 (m, 1H), 3.91 (q, *J* = 6.8 Hz, 1H), 3.59-3.53 (m, 1H), 3.43-3.33 (m, 3H), 1.93-1.85 (m, 2H), 1.67-1.59 (m, 2H), 1.57-1.48 (m, 6H), 1.39 (d, *J* = 6.8 Hz, 3H), 1.35-1.19 (m, 24H), 0.87 (t, *J* = 6.8 Hz, 6H).

### Step 6)

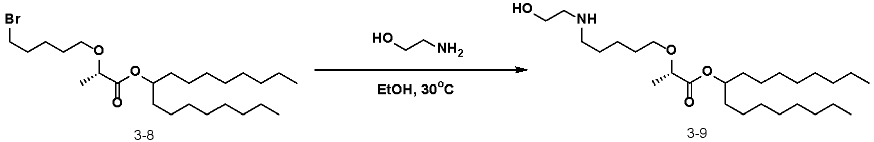

**3-8** (4.30 g, 9.00 mmol) was weighed out and dissolved in absolute EtOH (85 mL), and ethanolamine (16.5 g, 270 mmol) was added under a nitrogen atmosphere. The mixture was stirred overnight at 30 °C. A sample was taken and analyzed by LC-MS, and the results showed that the starting material was completely consumed. The solvent was removed by rotary evaporation under reduced pressure, and EA (200 mL) was added to the residue. The solution was washed twice with water (50 mL × 2) and twice with saturated brine (50 mL × 2), then dried over anhydrous sodium sulfate, and filtered. The filtrate was concentrated under reduced pressure and purified by normal-phase column chromatography (DCM:MeOH = 10:1) to give a colorless oil (3.30 g, yield: 80.1%).

MS: 458.4[M+H].

¹H NMR (400 MHz, CDCl₃) *δ* 4.96-4.88 (m, 1H), 3.90 (q, *J* = 6.8 Hz, 1H), 3.64 (t, *J* = 5.2 Hz, 2H), 3.57-3.51 (m, 1H), 3.38-3.31 (m, 1H), 2.77 (t, *J* = 4.8 Hz, 2H), 2.63 (t, *J* = 7.2 Hz, 2H), 1.65-1.57 (m, 2H), 1.56-1.48 (m, 6H), 1.44-1.27 (m, 5H), 1.26-1.19 (m, 24H), 0.86 (t, *J* = 6.8 Hz, 6H).

### Step 7)

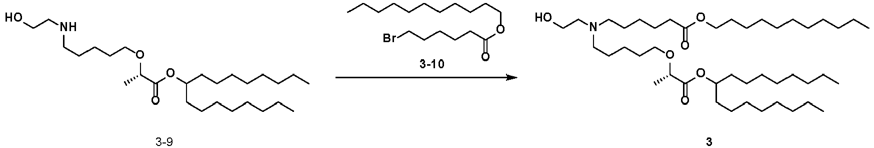

**3-9** (2.30 g, 5.00 mmol) was weighed out and dissolved in absolute EtOH (46 mL), and DIEA (1.90 g, 15.0 mmol) and **3-10** (3.50 g, 10 mmol) were added under a nitrogen atmosphere. The mixture was stirred overnight at 80 °C. A sample was taken and analyzed by LC-MS, and the results showed that most of the starting materials were consumed. The solvent was removed by rotary evaporation under reduced pressure, and EA (100 mL) was added to the residue. The solution was washed twice with water (50 mL × 2) and twice with saturated brine (50 mL × 2), then dried over anhydrous sodium sulfate, and filtered. The filtrate was concentrated under reduced pressure and purified by normal-phase column chromatography (DCM:MeOH = 15:1) to give a colorless oil (2.10 g, yield: 57.9%).

MS: 726.6[M+H].

¹H NMR (400 MHz, CDCl₃) *δ* 4.94-4.88 (m, 1H), 4.05 (t, *J* = 6.8 Hz, 2H), 3.90 (q, *J* = 6.8 Hz, 1H), 3.69-3.65 (m, 2H), 3.57-3.50 (m, 1H), 3.39-3.33 (m, 1H), 2.80-2.70 (m, 2H), 2.70-2.60 (m, 4H), 2.30 (t, *J* = 7.6 Hz, 2H), 1.65-1.20 (m, 61H), 0.89-0.85 (m, 9H).

### Example 4

### Step 1)

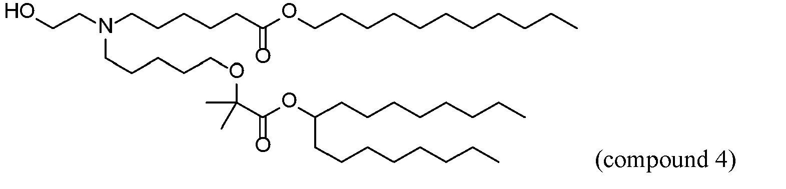

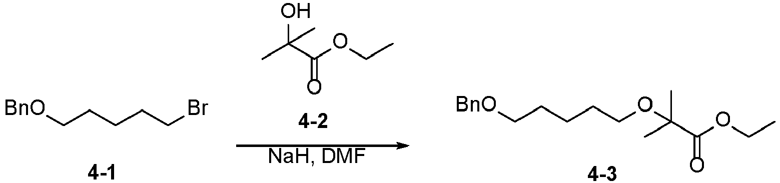

NaH (3.63 g, 0.091 mol) was weighed out and dissolved in DMF (200 mL), and the solution was cooled to 0 °C under an argon atmosphere. **4-2** (10 g, 0.076 mol) was added portionwise to the system, and the mixture was stirred at room temperature. **4-1** (19.5 g, 0.076 mol) was added to the reaction, and the mixture was stirred overnight at room temperature. 100 mL of water was added, and extraction was performed with ethyl acetate (0.5 L × 2). The organic phase was washed with saturated brine, then dried over anhydrous sodium sulfate, and filtered. The filtrate was concentrated under reduced pressure and purified by normal-phase column chromatography (PE:EA = 6:1) to give a colorless oil (5.30 g, yield: 22.3%).

MS: 309.2[M+H].

¹H NMR (400 MHz, CDCl₃) *δ* 7.40-7.25 (m, 5H), 4.50 (s, 2H), 4.18 (q, *J* = 7.2 Hz, 2H), 3.47 (t, *J* = 6.4 Hz, 2H), 3.36 (t, *J* = 6.8 Hz, 2H), 1.69-1.55 (m, 4H), 1.48-1.40 (m, 2H), 1.41 (s, 6H), 1.27 (t, *J* = 6.8 Hz, 3H).

### Step 2)

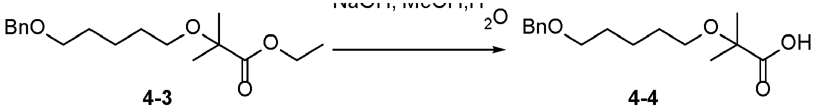

Compound **4-3** (5.20 g, 0.017 mol) was dissolved in MeOH (50 mL), and a solution of NaOH (2.69 g, 0.067 mol) in water (10 mL) was added under an ice-water bath. The mixture was stirred overnight at room temperature. The methanol was evaporated under reduced pressure, 100 mL of water was added, and the pH was adjusted to 3-4 with 1 N hydrochloric acid. Extraction was performed with ethyl acetate (400 mL × 2). The organic phase was washed with saturated brine, then dried over anhydrous sodium sulfate, and filtered. The filtrate was concentrated under reduced pressure to give a colorless oil (4.80 g, yield: 100%).

MS: 281.2[M+H].

¹H NMR (400 MHz, CDCl₃) *δ* 7.35-7.25 (m, 5H), 4.51 (s, 2H), 3.50-3.42 (m, 4H), 1.70-1.50 (m, 4H), 1.50-1.40 (m, 8H).

### Step 3)

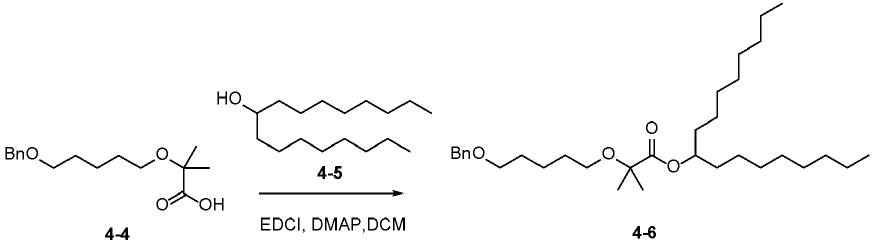

**4-4** (3.29 g, 0.012 mol) was weighed out and dissolved in DCM (100 mL), and **4-5** (2.70 g, 0.011 mol), EDCI (2.70 g, 0.014 mol), and DMAP (2.86 g, 0.023 mol) were added. The system was purged with argon gas, and the mixture was stirred overnight at room temperature. The reaction mixture was concentrated to dryness under reduced pressure, and the crude product was purified by normal-phase column chromatography (PE:EA = 50: 1) to give a colorless oil (4.70 g, yield: 85.7%).

¹H NMR (400 MHz, CDCl₃) *δ* 7.36-7.20 (m, 5H), 4.93-4.80 (m, 1H), 4.50 (s, 2H), 3.47 (t, *J* = 6.8 Hz, 2H), 3.37 (t, *J* = 6.8 Hz, 2H), 1.70-1.50 (m, 8H), 1.47-1.39 (m, 8H), 1.30-1.15 (m, 24H), 0.87 (t, *J* = 6.8 Hz, 6H).

### Step 4)

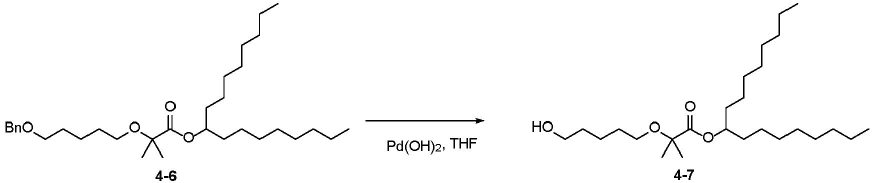

Compound **4-6** (4.50 g, 8.67 mmol) was dissolved in THF (90 mL), and Pd(OH)₂/C (0.90 g, 20 wt%) was added. The system was purged with a hydrogen balloon, and the mixture was stirred at room temperature for 5 h. The reaction mixture was concentrated to dryness under reduced pressure, and the crude product was purified by normal-phase column chromatography (PE:EA= 10:1) to give a colorless oil (3.19 g, yield: 85.7%).

¹H NMR (400 MHz, CDCl₃) *δ* 4.93-4.86 (m, 1H), 3.65 (t, *J* = 6.4 Hz, 2H), 3.38 (t, *J* = 6.4 Hz, 2H), 1.70-1.50 (m, 8H), 1.48-1.40 (m, 8H), 1.32-1.25 (m, 24H), 0.87 (t, *J* = 6.8 Hz, 6H).

### Step 5)

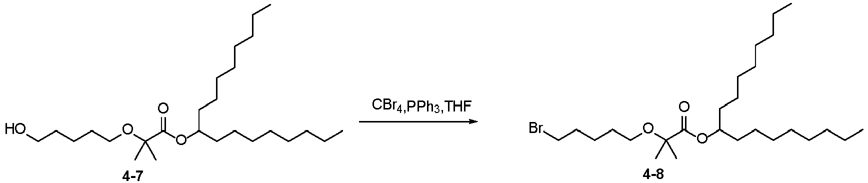

Compound **4-7** (1.50 g, 3.49 mmol) was dissolved in THF (30 mL), and CBr₄ (1.16 g, 3.49 mmol) and PPh₃ (0.92 g, 3.49 mmol) were sequentially added. The mixture was stirred overnight at room temperature under an argon atmosphere. A sample was taken and analyzed by TLC (PE:EA = 6:1), and the results showed that the starting material was completely consumed. Filtration was performed. The reaction mixture was concentrated to dryness under reduced pressure, and the crude product was purified by normal-phase column chromatography (PE:EA = 50:1) to give a colorless oil (1.50 g, yield: 87.2%).

¹H NMR (400 MHz, CDCl₃) *δ* 4.92-4.80 (m, 1H), 3.43-3.35 (m, 4H), 1.93-1.80 (m, 2H), 1.65-1.50 (m, 8H), 1.41 (s, 6H), 1.36-1.24 (m, 24H), 0.87 (t, *J* = 6.8 Hz, 6H).

### Step 6)

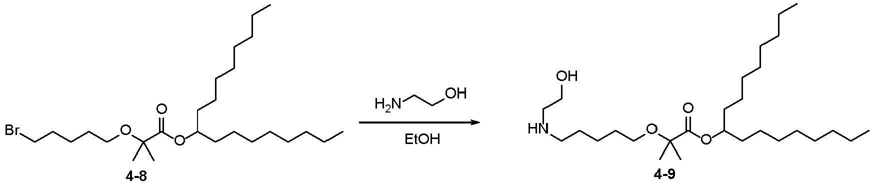

**4-8** (1.25 g, 2.55 mmol) was weighed out and dissolved in EtOH (15 mL), and ethanolamine (4.67 g, 76.5 mmol) was added. The mixture was stirred overnight at 25 °C under an argon atmosphere. Ethyl acetate (200 mL) was added. The mixture was washed with a saturated sodium chloride solution (100 mL × 2), dried over sodium sulfate, and filtered. The filtrate was concentrated and purified by normal-phase column chromatography (DCM:MeOH = 10: 1) to give a colorless oil (0.84 g, yield: 71.3%).

MS Calc: 471.4, Found: 472.4[M+H].

¹H NMR (400 MHz, CDCl₃) *δ* 4.91-4.80 (m, 1H), 3.68 (t, *J* = 4.8 Hz, 2H), 3.37 (t, *J* = 6.4 Hz, 2H), 2.83 (t, *J* = 5.2 Hz, 2H), 2.69 (t, *J* = 7.2 Hz, 2H), 1.62-1.52 (m, 8H), 1.45-1.36 (m, 8H), 1.55-1.24 (m, 24H), 0.87 (t, *J* = 6.8 Hz, 6H).

### Step 7)

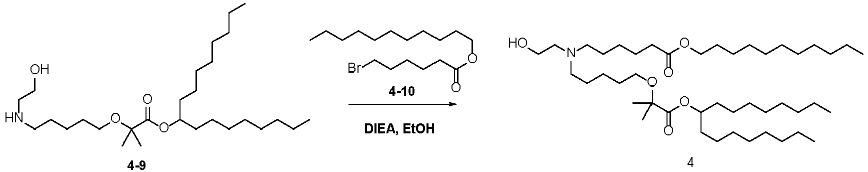

**4-9** (0.84 g, 1.78 mmol) was weighed out and dissolved in EtOH (15 mL), and **4-10** (0.81 g, 2.32 mmol) and DIPEA (0.46 g, 3.56 mmol) were added. The mixture was stirred overnight at 80 °C under an argon atmosphere. The reaction mixture was concentrated under reduced pressure and purified by normal-phase column chromatography (DCM:MeOH = 20:1) to give a colorless oil (0.61 g, yield: 46.2%).

MS: 740.7[M+H].

¹H NMR (400 MHz, CDCl₃) *δ* 4.90-4.86 (m, 1H), 4.05 (t, *J* = 6.8 Hz, 2H), 3.77 (s, 2H), 3.38 (t, *J* = 6.4 Hz, 2H), 2.90-2.70 (m, 6H), 2.31 (t, *J* = 7.2 Hz, 2H), 1.70-1.20 (m, 64H), 0.90-0.85 (m, 9H).

### Comparative Example 1

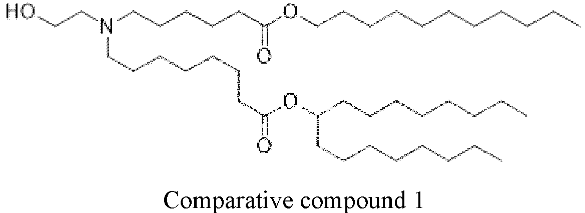

Obtained by the method in WO2017049245

### Test Example 1: Delivery Capacity of Lipid Particle Compositions

### 1.1. Preparation method

Compound 1, compound 2, and comparative compound 1 were each dissolved in an ethanol solution, and the resulting solution was mixed with solutions of DSPC, cholesterol, and DMG-PEG in ethanol in a mole ratio of 50:10:38.5:1.5 to prepare an ethanol lipid solution. mRNA encoding a human growth factor was dissolved in a citrate buffer to prepare an aqueous mRNA solution. Liposomes were prepared by mixing the ethanol lipid solution and the aqueous mRNA solution by microfluidics with a weight ratio of total lipids to mRNA of about 20:1. The ethanol was removed by dialysis against PBS solution to give an mRNA-encapsulating liposomal nanoparticle composition.

### 1.2. Lipid particle composition characterization

### Characterization method

The size and polydispersity index (PDI) of the liposomal nanoparticles were determined using a Malvern Zetasizer Nano ZS in a 173° backscatter detection mode through dynamic light scattering.

The liposome encapsulation efficiency was determined using a Quant-iT RiboGreen RNA assay kit.

The pKa of cations in the liposomal nanoparticles was determined using a fluorescence analysis based on 6-(p-toluidino)-2-naphthalenesulfonic acid sodium salt (TNS). 150 mM NaCl, 10 mM sodium phosphate, 10 mM sodium citrate, 10 mM sodium borate, and buffers of various pH levels ranging from 3 to 11.5 were prepared. A 300 µM TNS solution was prepared and added to the buffers. The lipid nanoparticles were added to the buffers of different pH levels and thoroughly mixed with them. Then the fluorescence intensity at an excitation wavelength of 325 nm and an emission wavelength of 435 nm was measured at room temperature using a fluorescence microplate reader. A fitting analysis of the fluorescence data was performed, with the pKa being the pH value at which half-maximal fluorescence intensity was produced. The relevant data are shown in Table 1.

**Table 1**

| No. | Size (nm) | PDI | Encapsulation efficiency (%) | pKa |
|---|---|---|---|---|
| Compound 1 | 87.8 | 0.08 | 94.6 | 6.65 |
| Compound 2 | 116.5 | 0.07 | 97.3 | 6.38 |
| Compound 3 | 79.6 | 0.09 | 96.7 | 6.49 |
| Compound 4 | 74.8 | 0.07 | 97.3 | 6.30 |
| Comparative compound 1 | 96.6 | 0.12 | 93.2 | 6.73 |

### 1.3. Measuring protein expression levels to evaluate the in vivo delivery capacity of the lipid particle compositions

To evaluate liposomal nanoparticles' effective *in vivo* delivery of mRNA and the expression of the corresponding encoded protein, liposomal nanoparticles with mRNA for expression of hepatocyte growth factor encapsulated therein were injected into the thigh muscles of 6-8 week old female BALB/c mice at a dose of 0.05 mg/kg. After 24 hours, the muscle tissue at the injection site was collected, ground, and lysed. The expression level of hepatocyte growth factor protein (pg/mg), i.e., the hepatocyte growth factor protein amount to which the unit muscle tissue total protein amount corresponds, was then measured using an ELISA kit. For the lipid nanoparticles to which each compound corresponds, at least 3 replicates were used, and the mean protein concentration was calculated.

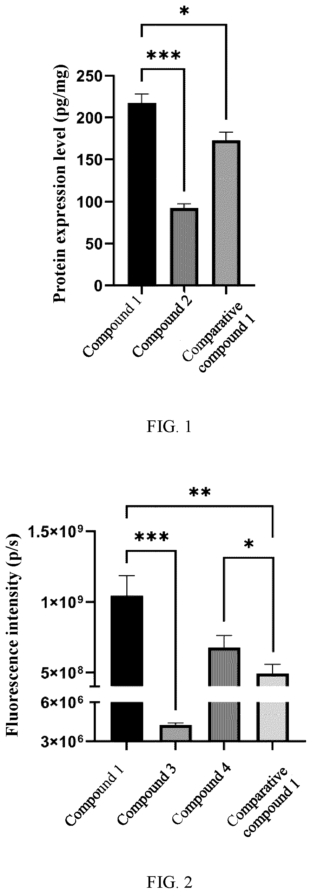

With the liposomal nanoparticles to which comparative compound 1 corresponds as a control, the intramuscular injection *in vivo* mRNA delivery efficiency of the liposomal nanoparticles to which compounds 1 and 2 correspond was measured. The relevant data are shown in FIG. 1 and Table 2.

In FIG. 1, * refers to statistical 0.01 < P < 0.05, indicating a statistically significant difference between groups; ** refers to statistical P < 0.01; and *** refers to statistical P < 0.001, indicating an extremely significant difference.

**Table 2**

| No. | Mean protein expression level (pg/mg) |
|---|---|
| Compound 1 | 217.3 |
| Compound 2 | 92.5 |
| Comparative compound 1 | 173.1 |

Conclusions: According to the protein expression levels shown in Table 2 and FIG. 1, the lipid nanoparticles to which compound 1 corresponds significantly outperformed those to which comparative compound 1 corresponds in delivering mRNA *in vivo* via intramuscular injection. Compound 1 also significantly outperformed compound 2. This indicates that the lipid nanoparticles to which compound 1 corresponds are capable of more efficiently delivering mRNA and enabling protein expression at a muscle site.

### Test Example 2: Evaluation of Tail Vein Injection In Vivo mRNA Delivery Efficiency of Lipid Particle Compositions

To evaluate liposomal nanoparticles' effective *in vivo* delivery of mRNA and the expression of the corresponding encoded protein, liposomal nanoparticles with mRNA for expression of luciferase encapsulated therein were injected into the tail veins of 6-8 week old female BALB/c mice at a dose of 0.5 mg/kg. After 6 hours, the luciferase substrate was injected intraperitoneally into each mouse. Fluorescence images of the mice were captured using an IVIS small animal optical *in vivo* imaging system (PerkinElme), and the whole-body fluorescence intensity of the mice was recorded. The level of fluorescence intensity reflects the expression level of luciferase protein, i.e., the *in vivo* mRNA delivery efficiency of the liposomal nanoparticles. For the lipid nanoparticles to which each compound corresponds, at least 3 biological replicates were used, and the mean fluorescence intensity was calculated. The data are shown in Table 3 and FIG. 2. In FIG. 2, the fluorescence intensity refers to the whole-body fluorescence intensity of the mice recorded using the IVIS small animal optical *in vivo* imaging system. The level of fluorescence intensity reflects the expression level of luciferase protein, i.e., the *in vivo* mRNA delivery efficiency of the lipid nanoparticles.

With the liposomal nanoparticles to which comparative compound 1 corresponds as a control, the mouse tail vein injection *in vivo* mRNA delivery efficiency of the liposomal nanoparticles to which compound 1, compound 3, and compound 4 correspond was measured.

In FIG. 2, * refers to statistical 0.01 < P < 0.05, indicating a statistically significant difference between groups; ** refers to statistical P < 0.01; and *** refers to statistical P < 0.001, indicating an extremely significant difference.

**Table 3**

| No. | Fluorescence intensity level (p/s) |
|---|---|
| Compound 1 | 1.05E+09 |
| Compound 3 | 4.28E+06 |
| Compound 4 | 6.78E+08 |
| Comparative compound 1 | 4.92E+08 |

Conclusions: According to the fluorescence intensity shown in Table 3 and FIG. 2, the lipid nanoparticles to which compound 1 corresponds exhibited significantly better tail vein injection *in vivo* mRNA delivery efficiency than those to which comparative compound 1 corresponds: the protein expression level of the lipid nanoparticles to which compound 1 corresponds was 2.1 times that of the lipid nanoparticles to which comparative compound 1 corresponds. Meanwhile, the lipid nanoparticles to which compound 1 corresponds significantly outperformed those to which comparative compound 3 corresponds, and the lipid nanoparticles to which compound 4 corresponds significantly outperformed those to which comparative compound 1 corresponds.

Test Example 3: Evaluation of Tissue Targeting in Lipid Nanoparticles' *In Vivo* Delivery To evaluate the targeting in liposomal nanoparticles' *in vivo* delivery, liposomal nanoparticles with mRNA for expression of hepatocyte growth factor encapsulated therein were injected into the tail veins of 6-8 week old female BALB/c mice at a dose of 0.5 mg/kg. Tissue samples were collected at 1 hour, 2 hours, 4 hours, 6 hours, 24 hours, and 48 hours after injection of the lipid nanoparticles, and the concentrations of cationic lipid molecules in the liver and spleen at the corresponding time points were determined by LC-MS mass spectrometry to evaluate the distribution of the lipid nanoparticles in different tissues. For the lipid nanoparticles to which each compound corresponds, at least 3 biological replicates were used, and the mean cationic lipid concentrations in the tissues were calculated.

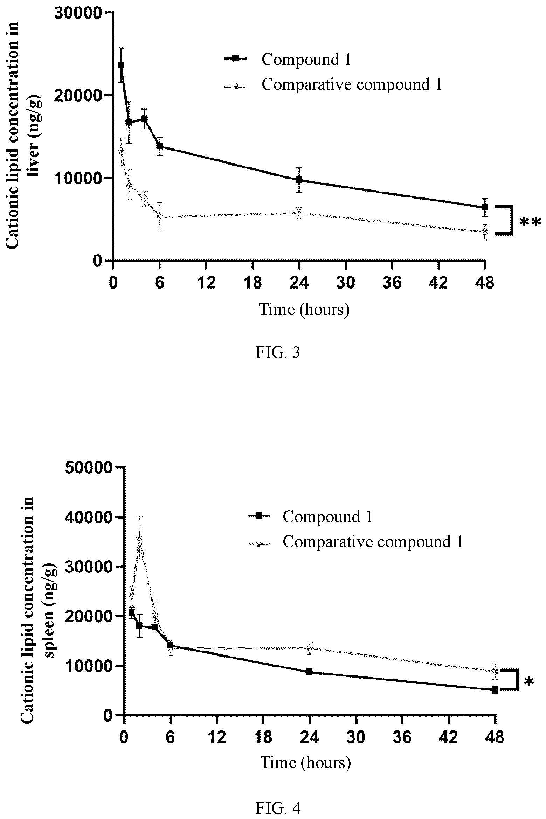

Conclusions: As shown in FIG. 3, in the samples taken from the liver at the various time points, the concentration of compound 1 was significantly higher than that of comparative compound 1. As shown in FIG. 4, in the spleen, the concentration of compound 1 was significantly lower than that of comparative compound 1. This indicates that the lipid nanoparticles to which compound 1 corresponds were distributed more in the liver and less in the spleen.

Based on pharmacokinetic parameters, the total cationic lipid concentrations in the tissues 48 hours after administration were calculated to evaluate the distribution of lipid nanoparticles in the liver and spleen. The table shows means calculated from multiple measurements. As shown in Table 4, the concentration of compound 1 was significantly higher than that of comparative compound 1 in the liver and significantly lower than that of comparative compound 1 in the spleen.

Therefore, the lipid nanoparticles to which compound 1 corresponds can target liver tissue better than those to which comparative compound 1 corresponds when delivering the nucleic acid.

In FIG. 3 and FIG. 4, * refers to statistical 0.01 < P < 0.05, indicating a statistically significant difference between groups; ** refers to statistical P < 0.01; and *** refers to statistical P < 0.001, indicating an extremely significant difference.

**Table 4**

| AUC₄₈ₕ(h*µg/mL) | Liver | Spleen |
|---|---|---|
| Compound 1 | 539.6 | 492.6 |
| Comparative compound 1 | 259.5 | 681.0 |

### Test Example 4: Evaluation of In Vivo Pharmacokinetics of Lipid Nanoparticles

To evaluate the targeting in liposomal nanoparticles' *in vivo* delivery, liposomal nanoparticles with mRNA for expression of hepatocyte growth factor encapsulated therein were injected into the tail veins of 6-8 week old female BALB/c mice at a dose of 0.5 mg/kg. Plasma, liver, and spleen samples were collected at 1 hour, 2 hours, 4 hours, 6 hours, 24 hours, and 48 hours after injection of the lipid nanoparticles, and the concentrations of cationic lipid molecules in the plasma samples at the corresponding time points were determined by LC-MS mass spectrometry to evaluate the metabolism and *in vivo* clearance rate of the lipid nanoparticles. For the lipid nanoparticles to which each compound corresponds, at least 3 biological replicates were used, and the mean cationic lipid concentrations in the tissues were calculated.

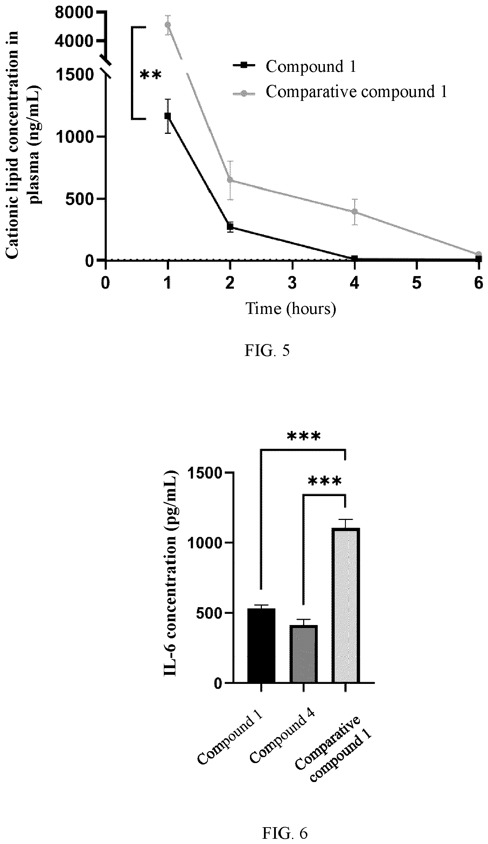

Conclusions: As shown in FIG. 5, the concentration of compound 1 in the 1-hour plasma sample was significantly lower than that of comparative compound 1: the concentration of compound 1 was 1163 ng/mL, and the concentration of comparative compound 1 was 6170 ng/mL; the residual concentration of comparative compound 1 was 5.3 times that of compound 1, indicating that the lipid nanoparticles to which compound 1 corresponds can be rapidly distributed to the target tissue, so that retention in the blood system is reduced. Compound 1 was completely cleared at 4 hours, while there was still a detectable concentration of comparative compound 1 at 6 hours. This indicates that compound 1 is cleared more rapidly from the blood system and metabolized better in the body.

Based on pharmacokinetic parameters, the half-lives of the cationic lipids in the tissues were calculated. The mean half-life of compound 1 in the liver was 34.2 hours, which was significantly lower than the half-life of comparative compound 1, which was 61.1 hours. The half-life of compound 1 in the spleen was 31.9 hours, which was significantly lower than that of comparative compound 1, which was 37.9 hours. This indicates that compound 1 was better degraded and metabolized in the tissues. The side effects caused by lipid nanoparticles in the human body, including inflammation and the like, are mainly due to cationic lipids. Compound 1 is metabolized and degraded more rapidly and thus is biologically safer than comparative compound 1.

In FIG. 5, * refers to statistical 0.01 < P < 0.05, indicating a statistically significant difference between groups; ** refers to statistical P < 0.01; and *** refers to statistical P < 0.001, indicating an extremely significant difference.

**Table 5**

| Half-life (hours) | Liver | Spleen |
|---|---|---|
| Compound 1 | 34.2 | 31.9 |
| Comparative compound 1 | 61.1 | 37.9 |

### Test Example 5: Evaluation of In Vivo Immunogenicity of Lipid Nanoparticles

When exogenous substances enter the bodies of mammals, they induce an innate immune response, thereby promoting the production of cytokines. These exogenous substances can cause inflammatory reactions after entry into the body, which easily give rise to adverse reactions such as fever and edema in the body. The immunogenicity of lipid nanoparticles in the body was therefore evaluated by evaluating the concentration of a cytokine such as interleukin 6 (IL-6) in the blood of mice injected with lipid nanoparticles. Lower cytokine concentrations indicate that the lipid nanoparticles have lower immunogenicity, which means that they are biologically safer.

Liposomal nanoparticles with mRNA for expression of the luciferase reporter gene encapsulated therein were injected into the tail veins of 6-8 week old female BALB/c mice at a dose of 0.5 mg/kg. After 6 hours, blood was collected and serum was isolated. The IL-6 concentration in the serum was measured using a mouse IL-6 ELISA kit. For the lipid nanoparticles to which each compound corresponds, at least 3 biological replicates were used, and the mean IL-6 concentration in the serum was calculated.

Conclusions: As shown in Table 6 and FIG. 6, the IL-6 concentrations in the serum of mice injected with the lipid nanoparticles to which compound 1 and compound 4 correspond were significantly lower than that of comparative compound 1. This indicates that the lipid nanoparticles to which compounds 1 and 4 correspond have lower immunogenicity in the body and are biologically safer than those to which comparative compound 1 corresponds.

In FIG. 6, * refers to statistical 0.01 < P < 0.05, indicating a statistically significant difference between groups; ** refers to statistical P < 0.01; and *** refers to statistical P < 0.001, indicating an extremely significant difference.

**Table 6**

| No. | Serum IL-6 concentration (pg/mL) |
|---|---|
| Compound 1 | 531.7 |
| Compound 4 | 412.8 |
| Comparative compound 1 | 1105.6 |

## Claims

1. A compound represented by formula I or a salt thereof, 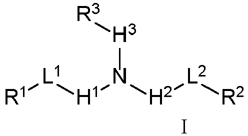
wherein L¹ and L² are each independently selected from the group consisting of -C(O)O-, -OC(O)-, -C(O)-, -OC(O)O-, -O-, -S(O)ₓ-, -S-S-, -C(O)S-, -SC(O)-, -NR^{a}C(O)-, -C(O)NR^{a}-, -NR^{a}C(O)NR^{a}-, -NR^{a}C(O)O-, -OC(O)NR^{a}-, or a bond, and R^{a} is selected from the group consisting of hydrogen or C₁₋₆ alkyl or C₂₋₆ alkenyl;
H¹ and H² are each independently selected from the group consisting of C₁₋₁₂ heteroalkylene, C₁₋₁₂ alkylene, and C₂₋₁₂ alkenylene, and at least one of H¹ and H² is C₁₋₁₂ heteroalkylene;
H³ is selected from the group consisting of C₁₋₂₄ alkylene, C₂₋₂₄ alkenylene, C₃₋₈ cycloalkylene, or C₃₋₈ cycloalkenylene;
R¹ and R² are each independently selected from the group consisting of C₁₋₂₄ alkyl or C₂₋₂₄ alkenyl;
R³ is selected from the group consisting of hydrogen, -CN, -C(O)OR⁴, -OC(O)R⁴, -OR⁵, or -NR⁵C(O)R⁴; R⁴ is selected from the group consisting of C₁₋₆ alkyl or C₂₋₆ alkenyl; R⁵ is selected from the group consisting of hydrogen, C₁₋₆ alkyl, or C₂₋₆ alkenyl;
x is selected from the group consisting of 0, 1, or 2.

2. The compound or the salt thereof according to claim 1, wherein H¹ is selected from the group consisting of C₁₋₁₂ heteroalkylene, preferably C₂₋₉ heteroalkylene.

3. The compound or the salt thereof according to claim 1 or 2, wherein L¹ and L² are each independently selected from the group consisting of -C(O)O-, -OC(O)-, or a bond.

4. The compound or the salt thereof according to any one of claims 1-3, wherein R¹ and R² are each independently selected from the group consisting of C₂₋₂₄ alkyl, preferably C₄-₁₈ alkyl; or R¹ and R² are each independently selected from the group consisting of C₂₋₂₄ alkenyl, preferably C₄-₁₈ alkenyl.

5. The compound or the salt thereof according to any one of claims 1-4, wherein the compound represented by formula I is 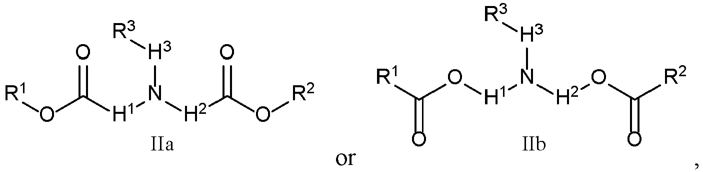 wherein H¹, H², H³, R¹, R², and R³ are as defined in claim 1.

6. The compound or the salt thereof according to any one of claims 1-5, wherein H³ is selected from the group consisting of C₂₋₂₄ alkenylene, C₃₋₈ cycloalkylene, or C₃₋₈ cycloalkenylene, preferably C₃₋₈ cycloalkylene; or H³ is selected from the group consisting of C₂₋₂₄ alkylene.

7. The compound or the salt thereof according to any one of claims 1-6, wherein the compound represented by formula I is 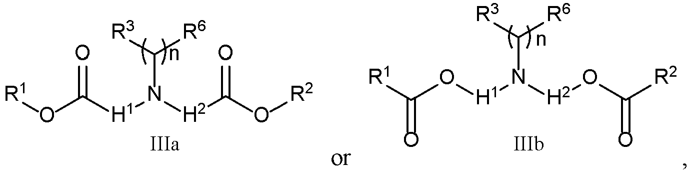 wherein each R⁶ is independently selected from the group consisting of hydrogen, hydroxy, C₁₋₆ alkyl, or C₂₋₆ alkenyl; n is selected from the group consisting of integers between 1 and 12, such as 2, 3, 4, 5, or 6; H¹, H², R¹, R², and R³ are as defined in claim 1.

8. The compound or the salt thereof according to any one of claims 1-7, wherein R³ is selected from the group consisting of -CN or hydroxy; or R³ is selected from the group consisting of -C(O)OR⁴, -OC(O)R⁴, or -NHC(O)R⁴, and R⁴ is as defined in claim 1; further, R⁴ is preferably C₁₋₆ alkyl, and is more preferably methyl, ethyl, or propyl.

9. The compound or the salt thereof according to claim 1 or 2, wherein the compound represented by formula I is 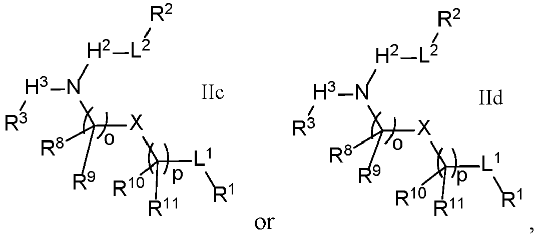 wherein X is -N(R⁷)- or -O-; o is selected from the group consisting of integers between 1 and 11, p is selected from the group consisting of integers between 1 and 5, and o + p is not greater than 12; R⁷ is selected from the group consisting of hydrogen, C₁₋₆ alkyl, or C₂₋₆ alkenyl; R⁸, R⁹, R¹⁰, and R¹¹ are each independently selected from the group consisting of hydrogen, hydroxy, C₁₋₆ alkyl, or C₂₋₆ alkenyl; L¹, L², H², H³, R¹, R², and R³ are as defined in claim 1.

10. The compound or the salt thereof according to claim 9, wherein p is selected from the group consisting of 1 or 2.

11. The compound or the salt thereof according to claim 1, wherein H² is selected from the group consisting of C₃₋₇ alkylene or C₃₋₇ alkenylene.

12. The compound or the salt thereof according to any one of claims 1-11, wherein the compound represented by formula I is 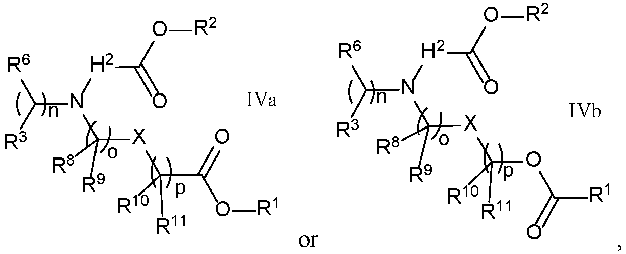 wherein H², R¹, R², and R³ are as defined in claim 1; R⁶ and n are as defined in claim 7; R⁸, R⁹, R¹⁰, R¹¹, X, o, and p are as defined in claim 9.

13. The compound or the salt thereof according to any one of claims 1-12, wherein R¹ is selected from the group consisting of:

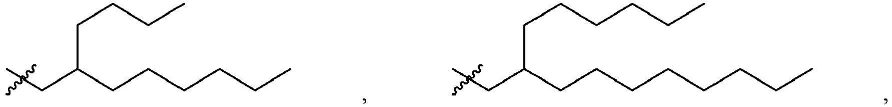

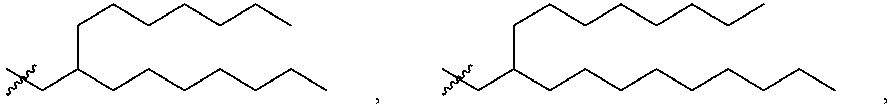

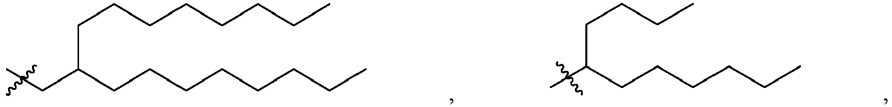

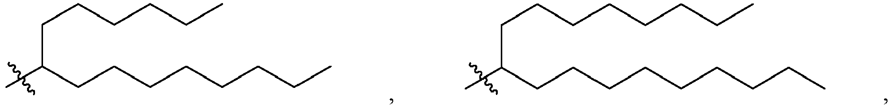

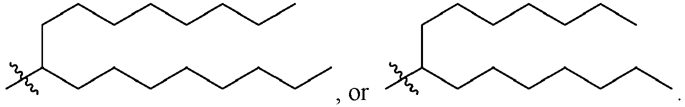

14. The compound or the salt thereof according to claim 1, wherein the compound represented by formula I is selected from the group consisting of

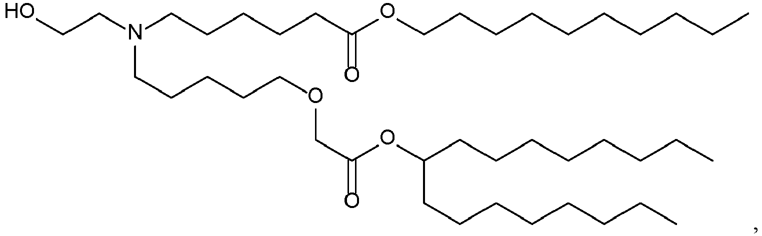

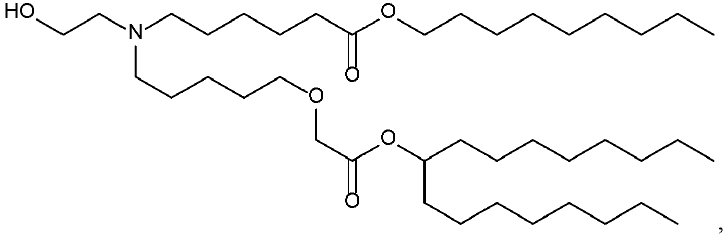

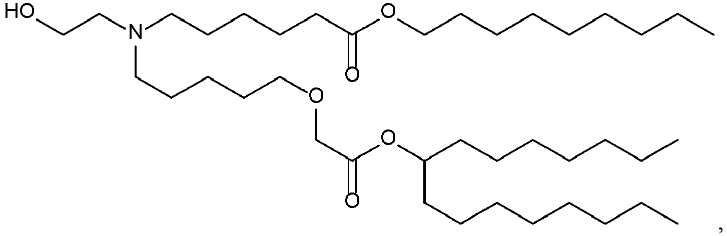

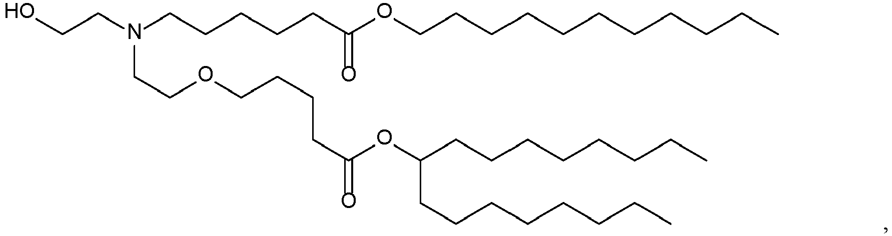

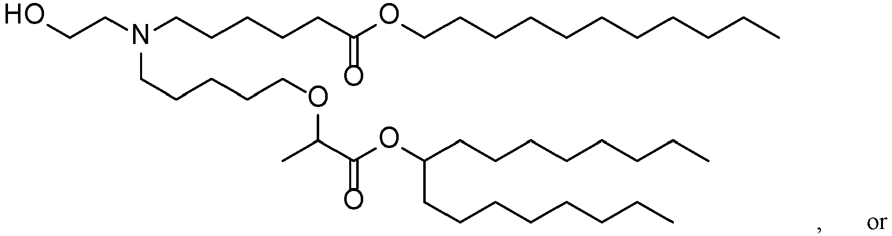

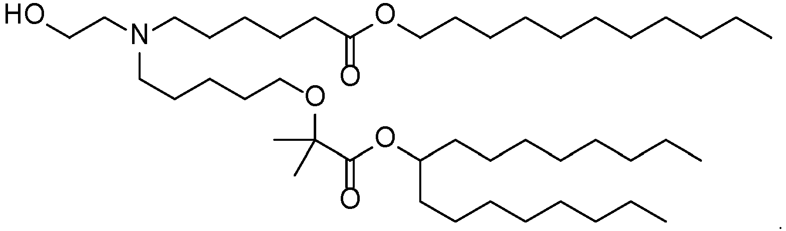

15. An isotopically substituted form of the compound according to any one of claims 1-14, wherein preferably, the isotopically substituted form is a form substituted with a deuterium atom.

16. A lipid particle, comprising the compound or the salt thereof according to any one of claims 1-15, and further comprising an active agent, wherein the active agent is preferably a polynucleotide or nucleic acid, such as an mRNA.

17. A pharmaceutical composition, comprising the lipid particle according to claim 15 or 16 and a pharmaceutically acceptable excipient.

18. Use of the compound or the salt thereof according to any one of claims 1-14, or the isotopically substituted form according to claim 15, or the lipid particle according to claim 16, or the pharmaceutical composition according to claim 17, in the preparation of a medicament for preventing and/or treating cancer, infection, autoimmune disease, neurodegenerative disease, and inflammation.
